# EUROPÄISCHE PATENTSCHRIFT

(11) **EP 2 288 372 B1**
(45) Veröffentlichungstag und Bekanntmachung des Hinweises auf die Patenterteilung: **08.02.2012**
(21) Anmeldenummer: 09741834.7
(22) Anmeldetag: 28.04.2009
(51) Int. Cl.: A61K 38/20, A61P 37/00, A61P 37/06

(54) **EIN IL-2 MUTEIN ZUR BEHANDLUNG ODER PROPHYLAXE EINER AUTOIMMUNERKRANKUNG**
AN IL-2 MUTANT FOR USE IN THE TREATMENT OR PROPHYLAXIS OF AUTOIMMUNE DISEASE
UN MUTANT DE L'INTERLEUKINE-2 POUR LE TRAITEMENT OU LA PROPHYLAXIE D'UNE MALADIE AUTO-IMMUNE

(30) Priorität: 08.05.2008 DE 102008023820
(43) Veröffentlichungstag der Anmeldung: 02.03.2011
(73) Patentinhaber: AiCuris GmbH & Co. KG, 42117 Wuppertal (DE)
(72) Erfinder: PAULSEN, Daniela, 42105 Wuppertal (DE); BRUNNER, Nina, 45279 Essen (DE); BRAY, Dorothy, Buckinghanshire HP144NN (GB)
(74) Vertreter: Witte, Weller & Partner
(86) Internationale Anmeldenummer: PCT/EP2009/003076
(87) Internationale Veröffentlichungsnummer: WO 2009/135615

(56) Entgegenhaltungen:
- WO-A1-99/60128
- WO-A2-03/015697
- US-B1- 6 348 192
- MATTHEWS L ET AL: "BAY 50-4798, a novel, high-affinity receptor-specific recombinant interleukin-2 analog, induces dose-dependent increases in CD25 expression and proliferation among unstimulated, human peripheral blood mononuclear cells in vitro" CLINICAL IMMUNOLOGY, ACADEMIC PRESS, US LNKD- DOI:10.1016/J.CLIM.2004.07.009, Bd. 113, Nr. 3, 1. Dezember 2004 (2004-12-01), Seiten 248-255, XP004609296 ISSN: 1521-6616
- TANG Q ET AL: "In Vitro-expanded Antigen-specific Regulatory T cells Suppress Autoimmune Diabetes" THE JOURNAL OF EXPERIMENTAL MEDICINE, ROCKEFELLER UNIVERSITY PRESS, UNITED STATES LNKD- DOI:10.1084/JEM.20040139, Bd. 199, Nr. 11, 7. Juni 2004 (2004-06-07) , Seiten 1455-1465, XP003005045 ISSN: 0022-1007
- NISHIMURA E ET AL: "Induction of antigen-specific immunologic tolerance by in vivo and in vitro antigen-specific expansion of naturally arising Foxp3+CD25+CD4+ regulatory t cells" INTERNATIONAL IMMUNOLOGY, OXFORD UNIVERSITY PRESS, GB LNKD- DOI:10.1093/INTIMM/DXH122, Bd. 16, Nr. 8, 5. Juli 2004 (2004-07-05), Seiten 1189-1201, XP003005849 ISSN: 0953-8178
- THORNTON ANGELA ET AL: "Activation requirements for the induction of CD4+CD25+ T cell suppressor function" EUROPEAN JOURNAL OF IMMUNOLOGY, WILEY - V C H VERLAG GMBH & CO. KGAA, DE LNKD- DOI:10.1002/EJI.200324455, Bd. 34, Nr. 2, 1. Februar 2004 (2004-02-01), Seiten 366-376, XP002407890 ISSN: 0014-2980
- MALEK THOMAS R ET AL: "CD4 regulatory T cells prevent lethal autoimmunity in IL-2Rbeta-deficient mice: Implications for the nonredundant function of IL-2" IMMUNITY, Bd. 17, Nr. 2, August 2002 (2002-08), Seiten 167-178, XP002579301 ISSN: 1074-7613
- ANTONY PAUL ANDREW ET AL: "CD4+CD25+ T regulatory cells, immunotherapy of cancer, and interleukin-2" JOURNAL OF IMMUNOTHERAPY, LIPPINCOTT WILLIAMS & WILKINS, HAGERSTOWN, MD, US LNKD- DOI:10.1097/01.CJI.0000155049.26787.45, Bd. 28, Nr. 2, 1. März 2005 (2005-03-01), Seiten 120-128, XP002457762 ISSN: 1524-9557
- BURCHILL ET AL: "Interleukin-2 receptor signaling in regulatory T cell development and homeostasis" IMMUNOLOGY LETTERS, ELSEVIER BV, NL LNKD- DOI:10.1016/J.IMLET.2007.08.005, Bd. 114, Nr. 1, 25. Oktober 2007 (2007-10-25), Seiten 1-8, XP022314034 ISSN: 0165-2478

## Beschreibung

Die vorliegende Erfindung betrifft ein Mittel zur Behandlung und/oder Prophylaxe einer Autoimmunerkrankung.

Autoimmunerkrankungen sind durch eine überschießende Reaktion des Immunsystems gegen körpereigenes Gewebe charakterisiert. Irrtümlicherweise erkennt das Immunsystem körpereigenes Gewebe als zu bekämpfenden Fremdkörper. Dadurch kommt es zu schweren Entzündungsreaktionen, die zu Schäden an hiervon betroffenen Organen führen.

Eine wichtige Rolle für die Unterscheidung zwischen körpereigenen und körperfremden Strukturen spielen T-Lymphocyten bzw. T-Zellen, die im Thymus "geschult" werden, nur an eigene Zelloberflächenmoleküle, die so genannten MHC-Moleküle, anzudocken und dabei körpereigene Strukturen zu tolerieren. Diese Prozesse werden "klonale Deletion" und "klonale Selektion" genannt. Bei der ersten Selektion im Thymus überleben nur diejenigen T-Zellen, die MHC-Moleküle auf den körpereigenen Zellmembranen erkennen können, wobei die Bindung jedoch nicht so fest ist, dass sie zur Aktivierung der T-Zellen führen könnte. T-Zellen, die eigene MHC-Moleküle gar nicht binden bzw. erkennen können, werden eliminiert. Bei der ebenfalls im Thymus stattfindenden klonalen Deletion werden diejenigen T-Zellen eliminiert, die körpereigene MHC-Moleküle derart "zielsicher" erkennen und fest binden können, dass sie aktiviert würden, was letztlich zur Zerstörung körpereigener Zellen führen würde. Dieser Prozess ist einer derjenigen Maßnahmen, die das Immunsystem ergreift, um das "Selbst" zu schonen und das "Fremd" bekämpfen zu können.

Bei Autoimmunkrankheiten verhält sich eine Gruppe der T-Zellen abweichend. Neben der immer noch funktionierenden Abwehr von Fremdmolekülen und -organismen greifen sie nun ebenfalls körpereigene Strukturen an. Organe oder Gewebe werden als fremd empfunden. Die Folgen können verschieden sein: Sofern lebensnotwendige Strukturen betroffen sind, wird eine Autoimmunkrankheit tödlich verlaufen. Das Immunsystem richtet seine Abwehr gegen diese Strukturen, zelluläre wie auch humorale Abwehrreaktionen werden in Gang gesetzt, Autoantikörper werden gebildet, was zur Folge hat, dass betroffene Organe im Laufe der Zeit ihre Funktion aufgeben. Zumeist wird das Immunsystem geschwächt, und der Körper wird anfällig für allerlei Krankheiten. Unter Umständen ist auch die Fremderkennung gestört, dadurch kann die Ausbreitung entarteter Krebszellen nicht mehr effektiv unterbunden werden, und die Betroffenen sind anfälliger für Infektionskrankheiten. Im Verlauf der Erkrankung zerstören Zellen des Immunsystems die körpereigenen Strukturen, während Reparaturmechanismen des Körpers nach Möglichkeit versuchen, die geschädigten Organteile zu erneuern. Dieser irrtümliche Angriff des Abwehrsystems setzt sich ohne Behandlung in der Regel lebenslang oder bis zur vollständigen Zerstörung der Zielstruktur fort.

Die genauen Ursachen von Autoimmunerkrankungen sind trotz intensiver Forschung weiterhin unklar. Anerkannte Hypothesen gehen davon aus, dass Autoimmunkrankheiten durch genetische Disposition, z.B. durch das Vorhandensein bestimmter MHC-Molekül-Varianten, in Kombination mit äußeren Einflüssen erworben werden. Gibt es im Körper des Betroffenen solche genetisch bedingten Faktoren, und es kommen darüber hinaus ungünstige Umweltfaktoren wie starker Stress, Infektionen, Schwangerschaft etc. hinzu, kann es zum Ausbruch von Autoimmunkrankheiten kommen.

Das Immunsystem besteht aus verschiedenen Zellen, die in der Lage sind, infektiöse Agenzien, die in dem Körper eingedrungen sind, zu bekämpfen. Der Mechanismus der Immunantwort umfasst die Aktivierung spezialisierter Zellen und die Akquisition von Effektorfunktionen, wie die Cytotoxizität bestimmter T-Zellen, die das so genannte CD8-Transmembranglycoprotein exprimieren und deshalb als CD8⁺-T-Zellen bezeichnet werden.

Regulatorische T-Zellen (T_{Reg}), früher auch als Suppressor-T-Zellen bezeichnet, sind eine spezialisierte Untergruppe der T-Zellen. Sie haben die Funktion, die Aktivierung des Immunsystems zu unterdrücken und dadurch die Selbsttoleranz des Immunsystems zu regulieren. Sie verhindern dadurch im gesunden Organismus die Entstehung von Autoimmunkrankheiten. Es wurden verschiedene T Reg-Populationen beschrieben, einschließlich jene, die die Proteine CD4, CD25 und Foxp3 exprimieren und deshalb als CD4⁺CD25⁺Foxp3⁺-T-Zellen bezeichnet werden. Darüber hinaus wurden T_{Reg} beschrieben, die zwar CD4 und Foxp3 exprimieren, nicht aber CD25, so genannte CD4⁺CD25⁻Foxp3⁺-T-Zellen.

Lan et al. (2005), Regulatory T cells: development, function and role in autoimmunity, Autoimmun. Rev. 4(6), S. 351 bis 363, beschreiben ein Mausmodell, in dem die Depletion von CD4⁺CD25⁺ Regulatorischen T-Zellen zur spontanen Entwicklung von Autoimmunerkrankungen führt.

Chatila T.A. (2005), Role of regulatory T cells in human diseases, 116(5), S. 949 bis 959, beschreiben, dass eine angeborene Defizienz von CD4⁺CD25⁺ Regulatorischen T-Zellen durch eine Mutation in dem Gen, das für das Protein Foxp3 codiert, zur Entwicklung von Autoimmunerkrankungen beiträgt.

Ein Überblick über Regulatorische T-Zellen findet sich in der Zeitschrift "Nature Immunology", die im März 2005 erschienen ist.

Autoimmunerkrankungen werden je nach betroffenem Organ behandelt. Grundprinzip der kausalen Therapie ist hierbei, die Aktivität des Immunsystems durch Gabe von Immunsuppressiva, z.B. Cortison, zu dämpfen. Diese Substanzen zeichnen sich durch mannigfaltige systemische Neben- und Wechselwirkungen aus, weshalb versucht wurde, neue Medikamente zu entwickeln, die spezifisch die am Krankheitsgeschehen beteiligten Mechanismen beeinflussen. Beispiele hierfür sind Natalizumab und Infliximab. Natalizumab ist ein monoklonaler Antikörper und selektiver Hemmstoff von IgG4, einem Adhäsionsmolekül, das sich an der Oberfläche von weißen Blutzellen befindet. Natalicumab hemmt das Einwandern von weißen Blutzellen in Entzündungsherde und wird zur Behandlung besonders aggressiver Formen von schubförmig verlaufender Multipler Sklerose eingesetzt. Infliximab ist ein chimärer monoklonaler Antikörper gegen den Tumor-Nekrose-Faktor α (TNFα), der eine Schlüsselrolle bei autoimmunen Entzündungsreaktionen spielt. Infliximab wird bei rheumatoider Arthritis, Morbus Crohn, Morbus Bechterew und Psoriasis eingesetzt.

In Ehrenstein et al. (2004), Compromised function of regulatory T cells in rheumatoid arthritis and reversal by anti-TNFα therapy, J. Exp. Med., Vol. 200, Nr. 3, S. 277-285, wird beschrieben, dass Infliximab als ein gegen TNFα gerichteter monoklonaler Antikörper die Therapie der rheumatoiden Arthritis verbessern kann.

Entsprechendes wird von Nadkarni et al. (2007), Anti-TNFα therapy induces a distinct regulatory T cell population in patients with rheumatoid arthritis via TGF-β, JEM Vol. 204, S. 33-39, vorgeschlagen.

Bresson et al. (2006) schlagen vor, Typ-I-Diabetes durch kombinierte Verabreichung eines Anti-CD3ε-spezifischen Antikörpers und eines Proinsulinpeptids zu behandeln.

Vandenbark et al. (2008), Therapeutic vaccination with a trivalent T-cell receptor (TCR) peptide vaccine restores deficient FoxP3 expression and TCR recognition in subjects with multiple sderosis, Immunology Vol. 123, S. 66-78, beschreiben eine Verbesserung der Kontrolle der autoreaktiven Antwort bei der Multiplen Sklerose nach Vaccinierung der Patienten mit bestimmten TCR-Peptiden.

Obwohl diese neueren Substanzen sehr spezifisch wirken, kann es zu schweren Nebenwirkungen kommen, z.B. dem Auftreten der progressiven multifokalen Leukenzephalopathie. Aus diesem Grunde wurde Natalizumab nur drei Monate nach seiner Erstzulassung in den USA wieder vom Markt genommen. Die Kosten für diese neuen Wirkstoffe sind sehr hoch. 300 mg Natalizumab kosten derzeit über EUR 2.000,-. 200 mg Infliximab kosten ca. EUR 1.700,-.

Vor diesem Hintergrund ist es eine Aufgabe der vorliegenden Erfindung, ein neues Arzneimittel zur Behandlung und/oder Prophylaxe einer Autoimmunerkrankung bereitzustellen, mit dem die Nachteile aus dem Stand der Technik möglichst vermieden werden. Insbesondere soll ein solches Arzneimittel bereitgestellt werden, das sich durch gute Verträglichkeit und niedrige Toxizität auszeichnet.

Diese Aufgaben werden durch die Bereitstellung eines Muteins von humanem Interleukin 2 (hIL-2-Mutein) gelöst, das entsprechend dem hIL-2-Wildtyp nummeriert ist und jeweils eine spezifische Aminosäuresubstitution an zumindest einer der Positionen 20, 88 oder 126 aufweist.

Die Erfinder haben überraschenderweise herausgefunden, dass ein solches hIL-2-Mutein hohes therapeutisches Potential aufweist, welches zur Behandlung und Prophylaxe von Autoimmunerkrankungen genutzt werden kann. So konnten sie bspw. in verschiedenen experimentellen Ansätzen demonstrieren, dass das hIL-2-Mutein in einem Lebewesen selektiv die Bildung von Regulatorischen T-Zellen, wie CD4⁺CD25⁺Foxp3⁺ und CD4⁺CD25-Foxp3⁺ induziert.

Überraschenderweise zeigt das erfindungsgemäße hIL-2-Mutein eine deutlich höhere Aktivität auf die Regulatorischen T-Zellen als hIL-2-Wildtyp. Dies zeigt sich besonders bei hohen Konzentrationen.

Für das erfindungsgemäße hIL-2-Mutein wird in der WO 99/60128 offenbart, dass es stärker an den dreikettigen IL-2-Rezeptor (IL-2Rαβγ) als an den zweikettigen IL-2 Rezeptor (IL-2Rβγ). Wie die Erfinder nun erstmals zeigen konnten, induziert das erfindungsgemäße hIL-2-Mutein gegenüber hIL-2-Wildtyp überraschenderweise jedoch ebenfalls verstärkt die Bildung von solchen Regulatorischen T-Zellen, denen die α-Untereinheit des IL-2-Rezeptors (CD25) fehlt (CD4⁺CD25-Foxp3⁺). Diese Subpopulation trägt zusätzlich dazu bei, die Aktivierung des Immunsystems zu unterdrücken und dadurch die Selbsttoleranz des Immunsystems zu regulieren. Das erfindungsgemäße hIL-2-Mutein weist dadurch wesentlich höhere Potenz als Wirkstoff zur Behandlung von Autoimmunerkrankungen auf als der hIL-2-Wildtyp.

Darüber hinaus weist das erfindungsgemäße hIL-2-Mutein gegenüber hIL-2-Wildtyp den weiteren Vorteil auf, dass es selektiv T-Zellen gegenüber natürlichen Killerzellen (NK-Zellen) aktiviert und dadurch ein reduziertes Toxizitätsprofil und einen erhöhten therapeutischen Index aufweist. Das erfindungsgemäße hIL-2-Mutein ist dadurch wesentlich besser verträglich als das hIL-2-Wildtyp; vgl. WO 99/60128.

Ferner konnte anhand der cytotoxischen CD3⁺CD8⁺CD45RO⁺-T-Zellen erstmals gezeigt werden, dass das erfindungsgemäße hIL-2-Mutein im Gegensatz zu dem hIL-2-Wildtyp überraschenderweise keinen oder nur einen geringen Effekt auf die Proliferation von CD8-positiven cytotoxischen T-Zellen hat, die auch als "naïve, central memory, early differentiated" und "late differentiated" CD8-T-Zellen bezeichnet werden. Dies ist in sofern von Vorteil, da die CD8⁺-cytotoxischen T-Zellen für persistente chronische Entzündungsprozesse bei Autoimmunerkrankungen verantwortlich gemacht werden; vgl. Liu et al. (2007), Multiple Sclerosis, 13, S. 149, und Haegeleet al. (2007), Neuroimmunol, 183, S. 168). Das erfindungsgemäße hIL-2-Mutein verhindert somit gegenüber dem hIL-2-Wildtyp eine weitere Verstärkung dieser durch die CD8⁺-T-Zellen verursachte Entzündungsreaktion, was einen weiteren Verträglichkeitsvorteil darstellt.

Wie die Erfinder ebenfalls zeigen konnten, stimuliert das erfindungsgemäße hIL-2-Mutein außerdem die antigenspezifische Aktivität der Immunzellen. Dies hat den Vorteil, dass mittels des hIL-2-Muteins selektiv krankheitsspezifische Immunzellen stimuliert werden und damit der systemische Effekt der Immuntherapie limitiert wird. Dadurch wird auch verhindert, dass mit der Verabreichung des hIL-2-Muteins andere Krankheiten induziert werden.

Weiterhin konnten die Erfinder anhand eines Mausmodells zum Diabetes mellitus Typ I zeigen, dass der Ausbruch einer Autoimmunerkrankung durch die Behandlung mit dem erfindungsgemäßen hIL-2-Mutein unterbunden werden kann.

Die der Erfindung zugrunde liegende Aufgabe wird hiermit vollkommen gelöst.

Erfindungsgemäß wird unter "Wildtyp" des humanen Interleukin 2 (hIL-2-Wildtyp) ein Polypeptid bzw. Protein verstanden, das die Aminosäuresequenz von 133 Aminosäuren aufweist, die in nativem humanen IL-2 vorliegt (ohne das Signalpeptid, das aus weiteren 20 N-terminalen Aminosäuren besteht). hIL-2-Wildtyp kann sowohl nativ als auch rekombinant exprimiert sein. Die Aminosäuresequenz von hIL-2-Wildtyp ist in Fujita et al. (1983), PNAS USA 80, S. 7437-7441 beschrieben, und zwar mit und ohne einem zusätzlichen N-terminalen Methionin, das notwendigerweise vorhanden ist, wenn das Protein in *E*. *coli* als intrazelluläre Fraktion exprimiert wird. Die Aminosäuresequenz des hIL-2-Widtyp ist im beiliegenden Sequenzprotokoll unter SEQ ID Nr. 1 angegeben. Die Nukleotidsequenz der cDNA, die für hIL-2 codiert, ist im beiliegenden Sequenzprotokoll unter SEQ ID Nr. 2 angegeben.

Erfindungsgemäß wird unter einem "Mutein" von humanem Interleukin 2 (hIL-2-Mutein) ein Polypeptid bzw. Protein verstanden, bei dem gegenüber dem hIL-2-Wildtyp spezifische Substitutionen vorgenommen wurden. Die Identifizierung der Positionen, an denen Substitutionen vorgenommen wurden, richtet sich nach den Positionen der Aminosäuren im hIL-2-Wildtyp, die beispielsweise der SEQ ID Nr. 1 zu entnehmen sind. Demnach findet sich an Position 1 ein Alanin (A), an Position 2 ein Prolin (P), an Position 133 ein Threonin (T) usw. Der Asparaginsäure-Rest (D) an der Position 20 ("D20") kann beispielsweise durch ein Isoleucin-Rest (I) oder ein Histidin (H) substituiert sein, so dass IL-2-Muteine gebildet werden, die als hIL-2-D20I bzw. hIL-2-D20H bezeichnet werden.

Es versteht sich, dass das erfindungsgemäße hIL-2-Mutein an mehreren der genannten Positionen 20, 88 oder 126 substituiert sein kann, so dass Kombinationsmutanten entstehen, die für die Behandlung einer Autoimmunerkrankung bzw. zur Induktion von Regulatorischen T-Zellen besonders geeignet sind.

Erfindungsgemäß umfasst ein hIL-2-Mutein auch ein modifiziertes Polypeptid, bspw. ein glycosyliertes hIL-2-Mutein. Glycosylierte hIL-2-Muteine sind bspw. in den US-Patentanmeldungen 09/310,026 und 10/051,657 offenbart.

Unter einem "Abschnitt" von hIL-2-Mutein wird ein solches Polypeptid verstanden, bei dem gegenüber dem hIL-2-Mutein N- und/oder C-terminal eine oder mehrere Aminosäuren fehlen, dieses aber gleichwohl noch ausreichend biologische Aktivität des hIL-2-Muteins aufweist, um erfindungsgemäß zur Behandlung und/oder Prophylaxe von Autoimmunerkrankungen eingesetzt zu werden. Diese Aktivität wird als ausreichend angesehen, wenn der Abschnitt zumindest 50%, vorzugsweise zumindest 60%, weiter bevorzugt zumindest 70%, weiter bevorzugt zumindest 80%, weiter bevorzugt zumindest 90% und höchst bevorzugt zumindest 95% der Aktivität des hIL-2-Muteins zur Induktion von Regulatorischen T-Zellen aufweist. Die Aktivität des hIL-2-Muteins lässt sich mittels dem Fachmann bekannter Verfahren einfach messen. Ein solches Verfahren ist bspw. in der WO 99/60128, dortige Beispiele 3 bis 5, offenbart.

Bei den Substitutionen an den genannten Positionen handelt es sich nicht um konservative Substitutionen, durch die eine Aminosäure gegen eine andere mit ähnlichen biochemischen Eigenschaften ausgetauscht wird.

Bei der Substitution an Position 20 handelt es sich nicht um eine solche, bei der die Asparaginsäure (D) gegen eine Glutaminsäure (E) ausgetauscht wird. Bei der Substitution an Position 88 handelt es sich nicht um eine solche, bei der das Asparagin (N) gegen ein Alanin (A), Prolin (P), Glycin (G), Glutamin (Q), Serin (S) oder Threonin (T) ausgetauscht ist. Ferner handelt es sich bei der Substitution an Position 126 nicht um eine solche, bei der das Glutamin (Q) gegen ein Alanin (A), Prolin (P), Glycin (G), Asparagin (N), Serin (S) oder Threonin (T) ausgetauscht ist. Diese Substitutionen würden die biologische Aktivität des hIL-2-Wildtyp nicht oder nur unwesentlich ändern.

An den genannten Positionen sind keine solchen Substitutionen vorgenommen, die Stellen für intermolekulare Quervernetzung oder inkorrekte Disulfidbrückenbindungen einbringen. Deshalb handelt es sich bei der Substitution des erfindungsgemäßen hIL-2-Mutein an Position 20 nicht um eine solche, bei der die Asparaginsäure (D) gegen Arginin (R), Asparagin (N), Asparaginsäure (D), Cystein (C), Glutaminsäure (E), Glycin (G), Leucin (L), Lysin (K), Phenylalanin (F), Prolin (P), Threonin (T) oder Tryptophan (W) ausgetauscht ist. Bei der Substitution an Position 88 handelt es sich nicht um eine solche, bei der das Asparagin (N) gegen Asparaginsäure (D), Cystein (C), Glutamin (Q), Tryptophan (W) oder Prolin (P) ausgetauscht ist. Bei der Substitution an Position 126 handelt es sich nicht um eine solche, bei der das Glutamin (Q) gegen ein Alanin (A), Histidin (H), Tryptophan (W), Cystein (C), Glutamin (Q), Glutaminsäure (E) oder Lysin (K) ausgetauscht ist.

Das erfindungsgemäße hIL-2-Mutein kann durch jede geeignete im Stand der Technik bekannte Methode hergestellt werden. Solche Methoden beinhalten das Konstruieren einer DNA-Sequenz, die für das erfindungsgemäße IL-2-Mutein der Erfindung codiert und beispielsweise die Nukleotidsequenz SEQ ID Nr. 2 umfasst und das Exprimieren dieser Sequenz in einem geeigneten Wirt. Diese Methode führt zu den erfindungsgemäßen Muteinen in rekombinanter Form. Allerdings kann das erfindungsgemäße Mutein auch durch chemische Synthese oder eine Kombination von chemischer Synthese und rekombinanter DNA-Technologie hergestellt werden. Die Herstellung des erfindungsgemäßen Muteins ist ausführlich in der WO 99/60128, dortige Ausführungsbeispiele 1 und 2, beschrieben.

Eines der spezifischen erfindungsgemäßen hIL-2-Muteine, bei dem an Position 88 das Asparagin (N) gegen ein Arginin (R) ausgetauscht ist (hIL-2-N88R), steht dem Fachmann unter der Bezeichnung BAY50-4798 zur Verfügung; vgl. Shanafelt et al. (2000), A T-cell-selective interleukin 2 mutein exhibits potent antitumor activity and is well tolerated in vivo, Nat. Biotechnol. Vol. 18, S. 1197-1202. Die Aminosäuresequenz von hIL-2-N88R ist im beiliegenden Sequenzprotokoll unter SEQ ID Nr. 3 angegeben.

Die Erkenntnisse der Erfinder waren insbesondere deshalb so überraschend, weil sich im Stand der Technik keinerlei Hinweise auf eine entsprechende Aktivität des IL-2-Muteins finden lassen.

So wird in der WO 99/60128 für das Mutein hIL-2-N88R offenbart, dass dieses selektiv T-Zellen gegenüber natürlichen Killerzellen aktivieren kann und in der Lage ist, die Metastasenbildung in der Lunge zu reduzieren.

In der WO 02/00243 wird eine stabile, histidinenthaltende, albuminfreie Formulierung für das Mutein hIL-2-N88R beschrieben.

In der US 2002/0164300 wird eine glycosylierte Variante des Muteins hIL-2-N88R beschrieben.

Die Verwendung des erfindungsgemäßen hIL-2-Muteins zur gezielten Behandlung und/oder Prophylaxe von Autoimmunerkrankungen oder zur selektiven Aktivierung von Regulatorischen T-Zellen in einem Lebewesen, wird im Stand der Technik weder beschrieben noch nahegelegt.

Selbst für das humane Wildtyp-IL-2 liegen entsprechende Erkenntnisse nicht vor.

Van der Vliet et al. (2007), Effects of the administration of high-dose interleukin-2 on immunoregulatory cell subsets in patients with advanced melanoma and renal cell cancer, Clin. Cancer Res. Vol. 13, S. 2100-2108, beschreiben, dass bei der Verabreichung von hohen Dosen von IL-2 dessen therapeutische Wirksamkeit zur Behandlung von Tumoren reduziert wird.

Ahmadzadeh und Rosenberg (2006), IL-2 administration increases CD4+CD25h1Foxp3+ regulatory T cells in cancer patients, Blood, Vol. 107, S. 2409-2414, schlagen vor, die therapeutische Wirksamkeit von humanem Wildtyp-IL-2 in Tumorpatienten dadurch zu verbessern, indem die Regulatorischen T-Zellen der Patienten eliminiert werden.

Dieser Ansatz stellte sich jedoch nicht als erfolgversprechend heraus; vgl. Powell et al. (2007), Inability to mediate prolonged reduction of regulatory T cells after transfer of autologous CD25-depleted PBMC and interleukin-2 after lymphodepleting chemotherapy, J. Immunother. Vol. 30, S. 438-447.

Antony and Restifo (2005), CD4+CD25+ T regulatory cells, immunotherapy of cancer, and interleukin-2, J. Immunother. Vol. 28, S. 120-128, lehnen IL-2 als Immuntherapeutikum eher ab und beschreiben sogar, dass die Verabreichung von IL-2 Autoimmunität induzieren kann.

In die gleiche Stoßrichtung zielen Knoechel et al. (2005), Sequential development of interleukin 2-dependent effector and regulatory T cells in response to endogenous systemic antigen, JEM Vol. 202, S. 1375-1386, und schlagen sogar einen IL-2-Antagonismus, d.h. eine Hemmung von IL-2-Mechanismen vor, um die Frühphase von Autoimmunerkrankungen zu behandeln.

Im Stand der Technik finden sich deshalb keine Anhaltspunkte, die die erfindungsgemäße Lösung nahelegen.

So haben die Erfinder auch erkannt, dass die therapeutischen Effekte von hIL-2-Mutein je nach Indikation und eingesetzter Konzentration unterschiedlich sein können. Eine hohe Konzentration von hIL-2 kann zur Behandlung von Autoimmunerkrankungen vorteilhaft, in der Therapie von Tumorerkrankungen jedoch contraindiziert sein.

Bei der erfindungsgemäßen Verwendung ist durch die Substitution an Position 88 ein Asparagin gegen ein Arginin (hIL-2-N88R), oder gegen ein Glycin (hIL-2-N88G), oder gegen ein Isoleucin (hIL-2-N88I), und/oder durch die Substitution an Position 20 eine Asparaginsäure gegen ein Histidin (hIL-2-D20H), oder gegen ein Isoleucin (hIL-2-D20I), oder gegen ein Tyrosin (hIL-2-D20Y), oder durch die Substitution an Position 126 ein Glutamin gegen ein Leucin (hIL-2-Q126L) ausgetauscht.

Diese Maßnahme hat den Vorteil, dass ein solches erfindungsgemäßes hIL-2-Mutein Verwendung findet, das sich dadurch auszeichnet, dass es besonders selektiv T-Zellen gegenüber natürlichen Killerzellen aktiviert und dadurch ein hohes therapeutisches Potential und niedrige Toxizität aufweist. Diese Eigenschaften der bevorzugten erfindungsgemäßen hIL-2-Muteine werden beschrieben in der WO 99/60128.

Erfindungsgemäß ist es bevorzugt, wenn das hIL2-Mutein zumindest eine weitere Aminosäuresubstitution an einer beliebigen Position, ausgenommen die Positionen 20, 88 oder 126, aufweist, so dass das derart weiter substituierte hIL-2-Mutein eine Aminosäuresequenz aufweist, die zumindest zu 80%, vorzugsweise zu 85%, weiter bevorzugt zu 90%, weiter bevorzugt zu 95%, höchst bevorzugt zu 99% identisch ist mit der Aminosäuresequenz des hIL-2-Muteins, das gegenüber dem hIL-2-Wildtyp, außer an zumindest einer der Positionen 20, 88 oder 126, nicht weiter substituiert ist.

Diese Maßnahme hat den Vorteil, dass alternative Primärstrukhuen bereitgestellt werden, die ggf. einfacher zu synthetisieren sind, als das hIL-2-Mutein, das außer an zumindest einer der Positionen 20, 88 oder 126 ansonsten dem hIL-2-Wildtyp entspricht. Um ein Polypeptid mit der biologischen Aktivität des hIL-2-Muteins und damit ein Arzneimittels zur Behandlung und/oder Prophylaxe einer Autoimmunerkrankung zu erhalten, ist es nicht zwingend erforderlich, ein Polypeptid bereitzustellen, das eine Aminosäuresequenz aufweist, die 100% identisch ist mit der Aminosäuresequenz des erfindungsgemäßen hIL-2-Muteins. Es ist vielmehr ausreichend, wenn hinreichend hohe Identität gegeben ist, wobei gegebenenfalls moderate Aktivitätseinbußen tolerabel sind, vorzugsweise aber mindestens 50%, 60%, 70%, 80%, 90%, 95% bzw. 99% der Aktivität erhalten bleibt. Die angegebenen Identitäten beziehen sich auf einen Abschnitt des erfindungsgemäßen hIL-2-Muteins mit ≥ 10 Aminosäuren. Der Grad der Homologie lässt sich leicht mittels dem Fachmann bekannter Verfahren ermitteln, beispielsweise einer BLAST-Analyse oder mittels des MegAlign-Moduls des Lasergene-Programms von DNAStar Inc.

Erfindungsgemäß ist es ferner bevorzugt, wenn es sich bei der weiteren Aminosäuresubstitution an einer beliebigen Position, ausgenommen die Positionen 20, 88 oder 126, um eine konservative Aminosäuresubstitution handelt.

Diese Maßnahme hat den Vorteil, dass weitere Varianten des erfindungsgemäßen hIL-2-Muteins bereitgestellt werden, die eine ausreichend hohe Aktivität zur Behandlung und/oder Prophylaxe von Autoimmunerkrankungen bzw. zur Induktion von Regulatorischen T-Zellen in einem Lebewesen aufweisen. Es ist dem Fachmann bekannt, dass konservative Substitutionen keinen oder lediglich einen minimalen Effekt auf die Sekundär- oder Tertiärstruktur des Muteins haben. Solche konservativen Substitutionen umfassen jene, die von Dayhoff in "The Atlas of Protein Sequence and Structure. Vol. 5", Natl. Biomedical Research, beschrieben sind. Beispielsweise können Aminosäuren, die zu einer der folgenden Gruppen gehören, gegeneinander ausgetauscht werden, d.h. bilden einen konservativen Austausch:
- Alanin (A), Prolin (P), Glycin (G), Asparagin (N), Serin (S), Threonin (T);
- Cystein (C), Serin (S), Tyrosin (Y), Threonin (T);
- Valin (V), Isoleucin (I), Leucin (L), Methionin (M), Alanin (A), Phenylalanin (F);
- Lysin (K), Arginin (R), Histidin (H);
- Phenylalanin (F), Tyrosin (Y), Tryptophan (W), Histidin (H); und
- Asparaginsäure (D), Glutaminsäure (E).

Bei dem Mittel zur Induktion der Bildung von Regulatorischen T-Zellen in einem Lebewesen handelt es sich vorzugsweise um ein Arzneimittel, das einen pharmazeutisch akzeptablen Träger aufweist.

Diese Maßnahme hat den Vorteil, dass das Mittel bereits in einer solchen Form bereitgestellt wird, die eine direkte Applikation in das Lebewesen, vorzugsweise einen Menschen, ermöglicht.

Pharmazeutisch akzeptable Träger sind im Stand der Technik umfassend beschrieben; vgl. Row et al. (2006), Handbook of Pharmaceutical Excipients, 5. Auflage, Pharmaceutical Press and American Pharmasists' Association; Bauer et al. (1999), Lehrbuch der pharmazeutischen Technologie, Wissenschaftliche Verlagsgesellschaft mbH Stuttgart. Eine besonders bevorzugte Formulierung ist jene, die in der WO 02/00243 offenbart wird. Diese Formulierung ist albuminfrei und die Stabilisierung des hIL-2-Muteins bzw. Abschnittes hiervon erfolgt mit Histidin. Vorzugsweise weist das fertige Arzneimittel folgende Bestandteile in folgenden Konzentrationen auf: hIL-2-Mutein oder eine Abschnitt hiervon = 0,1-5 mg/ml; Histidin = 0,08-1,6 Gew.-%; NaCl = 0-0,9 Gew.-%; Saccharose = 1-10 Gew.-%; Glycin = 0-0,3 Gew.-%, und weist einen pH-Wert von ca. 5 bis 6,5 auf.

Nach einer besonderen Ausgestaltung weist das Arzneimittel zusätzlich ein Immunsuppressivum auf.

Das Arzneimittel kann zur Behandlung und/oder Prophylaxe von Autoimmunerkrankungen aufgrund der besonderen Potenz des erfindungsgemäßen hIL-2-Muteins bereits als Monopräparat eingesetzt werden. Ein solches Monopräparat weist das erfindungsgemäße hIL-2-Mutein als alleinigen Wirkstoff auf. Pharmazeutisch akzeptable Träger, Lösungsmittel (Puffer, Wasser etc.), Hilfsstoffe etc. sind in diesem Zusammenhang keine Wirkstoffe.

Diese Maßnahme hat den Vorteil, dass der therapeutische Index des erfindungsgemäßen Arzneimittels durch Hinzunahme eines klassischen Immunsuppressivums nochmals erhöht wird.

Bevorzugt ist es, wenn das Immunsuppressivum ausgewählt ist aus der Gruppe bestehend aus Glucocortikoid, einschließlich Decortin, Prednisol; Azathioprin; Cyclosporin A; Mykophenolatmofetil; Tacrolimus; Anti-T-Lymphocytenglobulin, Anti-CD3-Antikörper, einschließlich Muromonab; Anti-CD25-Antikörper, einschließlich Basiliximab und Daclizumab; Anti-TNF-α-Antikörper, einschließlich Infliximab und Adalimumab; Azathioprin; Methotrexat; Ciclosporin; Sirolimus; Everolimus; Fingolimod; CellCept; Myfortic; Cyclophosphamid.

Diese Maßnahme hat den Vorteil, dass ein solches Immunsuppressivum eingesetzt wird, das nachweislich therapeutische Wirksamkeit bei Autoimmunerkrankungen aufweist und im Stand der Technik ausreichend zur Verfügung steht.

Weiter ist es bevorzugt, wenn die Autoimmunerkrankung ausgewählt ist aus der Gruppe bestehend aus: Diabetes-mellitus-Typ I, Rheumatoide Arthritis, Multiple Sklerose, Chronische Gastritis, Morbus Crohn, Morbus Basedow, Morbus Bechterew, Psoriasis, Myasthenia gravis, Autoimmunhepatitis, APECED, Chrug-Strauss-Syndrom, Colitis ulcerosa, Glomerulonephritis, Guillain-Barré-Syndrom, Hashimoto-Thyreoiditis, Lichen sclerolus, systemischer Lupus erythematodes, PANDAS, Rheumatisches Fieber, Sarkoidose, Sjörgren-Syndrom, Stiff-Man-Syndrom, Sklerodermie, Wegenersche Granulomatose, Vitilogo, Autoimmunenteropathie, Goodpasture-Syndrom, Dermatomyositis, Polymyositis, Autoimmunallergie, Asthma und Autoimmunreaktion nach Organtransplantationen.

Diese Maßnahme hat den Vorteil, dass ein solches Arzneimittel bereitgestellt wird, das zur Behandlung und/oder Prophylaxe der wichtigsten Autoimmunerkrankungen eingesetzt werden kann.

Ein weiterer Gegenstand der vorliegenden Erfindung betrifft ein Arzneimittel zur Behandlung und/oder Prophylaxe einer Autoimmunerkrankung, das das erfindungsgemäße hIL-2-Mutein aufweist.

Die im Zusammenhang mit der erfindungsgemäßen Verwendung beschriebenen Eigenschaften und Vorteile sowie Definitionen gelten gleichermaßen für das erfindungsgemäße Arzneimittel.

Offenbart wird ferner ein Mittel zur Bildung von Regulatorischen T-Zellen (T_{Reg}) in einem Lebewesen, das das erfindungsgemäße hIL-2-Mutein oder einen Abschnitt hiervon aufweist.

Die Vorteile und Eigenschaften sowie Definitionen der erfindungsgemäßen Verwendung gelten entsprechend für das erfindungsgemäße Mittel.

Offenbart werden außerdem Verfahren zur Behandlung und/oder Prophylaxe einer Autoimmunerkrankung in einem Lebewesen und zur Bildung von Regulatorischen T-Zellen (T_{Reg}) in einem Lebewesen, die jeweils die folgenden Schritte aufweisen: (a) Bereitstellen eines Muteins von humanem Interleukin 2 (hIL-2-Mutein) oder eines Abschnittes hiervon, (b) Verabreichung des hIL-2-Muteins oder des Abschnittes hiervon in ein Lebewesen, und (c) gegebenenfalls Wiederholung der Schritte (a) und (b), wobei es sich bei dem hIL-2-Mutein oder den Abschnitt hiervon um das erfindungsgemäße hIL-2-Mutein oder einen Abschnitt hiervon handelt.

Bei dem Lebewesen handelt es sich vorzugsweise um ein Säugetier, weiter bevorzugt um ein menschliches Lebewesen.

Die im Zusammenhang mit der erfindungsgemäßen Verwendung beschriebenen Eigenschaften und Vorteile sowie Definitionen gelten gleichermaßen für die vorstehend genannten Verfahren zur Behandlung und/oder Prophylaxe einer Autoimmunerkrankung in einem Lebewesen und zur Bildung von Regulatorischen T-Zellen (T_{Reg}) in einem Lebewesen.

Offenbart wird außerdem ein Verfahren zur Bildung von Regulatorischen T-Zellen (T_{Reg}) *in vitro,* das folgende Schritte aufweist: (a) Bereitstellung eines Muteins von humanem Interleukin-2 (hIL-2-Mutein) oder eines Abschnittes hiervon, (b) Inkontaktbringen des hIL-2-Muteins oder des Abschnittes hiervon mit peripheren mononuklearen Blutzellen (PBMCs), und (c) ggf. Wiederholung der Schritte (a) und (b), wobei es sich bei dem hIL-2-Mutein oder den Abschnitt hiervon um das erfindungsgemäße hIL-2-Mutein oder einen Abschnitt hiervon handelt.

Das Inkontaktbringen von hIL-2 bzw. den Abschnitten hiervon mit den PBMCs kann in jedem geeigneten Medium zur Kultivierung der PBMCs erfolgen.

Die im Zusammenhang mit der erfindungsgemäßen Verwendung beschriebenen Eigenschaften und Vorteile sowie Definitionen gelten gleichermaßen für das vorstehend genannte Verfahren zur Bildung von Regulatorischen T-Zellen (T_{Reg}) *in vitro.*

Offenbart wird ferner ein Verfahren zur Behandlung und/oder Prophylaxe einer Autoimmunerkrankung in einem Lebewesen, das folgende Schritte aufweist: (a) Bereitstellung eines Muteins von humanem Interleukin-2 (hIL-2-Mutein) oder eines Abschnittes hiervon, (b) Inkontaktbringen des hIL-2-Muteins oder des Abschnittes hiervon mit aus einem ersten Lebewesen stammenden peripheren mononuklearen Blutzellen (PBMCs), (c) Inkubation des hIL-2-Muteins oder des Abschnittes hiervon mit den PBMCs, um eine Zellpopulation, die Regulatorische T-Zellen (T_{Reg}) aufweist, zu erhalten, und (d) Zufuhr der Zellpopulation in ein zweites Lebewesen, wobei es sich bei dem hIL-2-Mutein oder den Abschnitt hiervon um das erfindungsgemäße hIL-2-Mutein oder einen Abschnitt hiervon handelt.

Das erste Lebewesen und das zweite Lebewesen weisen vorzugsweise die identische Blutgruppe auf, wobei es besonders bevorzugt ist, wenn es sich bei dem ersten und dem zweiten Lebewesen um identische Lebewesen bzw. Individuen handelt.

Hierbei ist von Vorteil, dass es bei der Zufuhr bzw. Reinfusion der Zellpopulation zu keinen unerwünschten Immunreaktionen gegen die Zellen kommt und das Verfahren deshalb besonders nebenwirkungsarm ist.

Die im Zusammenhang mit der erfindungsgemäßen Verwendung beschriebenen Eigenschaften und Vorteile sowie Definitionen gelten gleichermaßen für das vorstehend genannte Verfahren zur Behandlung und/oder Prophylaxe einer Autoimmunerkrankung in einem Lebewesen.

Im Folgenden wird die Erfindung anhand von Ausführungsbeispielen näher erläutert, die rein exemplarischen Charakter haben und die Reichweite der Erfindung nicht einschränken. Dabei wird Bezug auf die beigefügten Figuren genommen, in denen Folgendes dargestellt ist:
- Fig. 1: zeigt, dass hIL-2-N88R in gesunden Probanden bei gleicher oder geringerer Dosierung im Vergleich zu Proleukin einen größeren Anstieg der Regulatorischen CD4⁺CD25⁺Foxp3⁺-T-Zellen induziert;
- Fig. 2: zeigt, dass hIL-2-N88R in gesunden Probanden bei gleicher oder geringerer Dosierung im Vergleich zu Proleukin einen größeren Anstieg der Regulatorischen CD4⁺CD25⁻Foxp3⁺-T-Zellen induziert;
- Fig. 3: zeigt, dass hIL-2-N88R in Melanompatienten bei gleicher oder geringerer Dosierung im Vergleich zu Proleukin einen größeren Anstieg der Regulatorischen CD4⁺CD25⁺Foxp3⁺-T-Zellen induziert;
- Fig. 4: zeigt, dass hIL-2-N88R in Melanompatienten bei gleicher oder geringerer Dosierung im Vergleich zu Proleukin einen größeren Anstieg der Regulatorischen CD4⁺CD25⁻Foxp3⁺T-Zellen induziert;
- Fig. 5: zeigt, dass hIL-2-N88R in Multiple-Sklerose-Patienten bei gleicher oder geringerer Dosierung im Vergleich zu Proleukin einen größeren Anstieg der Regulatorischen CD4⁺CD25⁺Foxp3⁺-T-Zellen induziert;
- Fig. 6: zeigt, dass hIL-2-N88R in Multiple-Sklerose-Patienten bei gleicher oder geringerer Dosierung im Vergleich zu Proleukin einen größeren Anstieg der Regulatorischen CD4⁺CD25⁻Foxp3⁺-T-Zellen induziert;
- Fig. 7: zeigt, dass hIL-2-N88R in Multiple-Sklerose-Patienten bei gleicher oder höherer Dosierung im Vergleich zu Proleukin einen geringeren Anstieg der Cytotoxischen CFSElow/CD3⁺CD8⁺CD45RO⁺-T-Zellen induziert;
- Fig. 8: zeigt, dass hIL-2-N88R in gesunden Probanden bei gleicher oder höherer Dosierung im Vergleich zu Proleukin einen geringeren Anstieg der Cytotoxischen CFSElow/CD3⁺CD8⁺CD45RO⁺-T-Zellen induziert;
- Fig. 9: zeigt, dass hIL-2-N88R im Maus-Diabetes-Typ-I-Modell im Vergleich zu hIL-2-Wildtyp zu einem höheren prozentualen Anstieg von FoxP3⁺-Zellen innerhalb der CD4⁺-Zellen führt (A). Diese CD4⁺FoxP3⁺-Zellen weisen außerdern eine höhere Expression von CD25 auf (B).
- Fig. 10: zeigt, dass hIL-2-N88R im Maus-Diabetes-Typ-I-Model im Gegensatz zu hIL-2-Wildtyp die Entwicklung der Diabetes unterbindet.

### Ausführungsbeispiele

### 1. Material und Methoden

### 1.1 Abtrennung von PBMCs von Vollblut zur Verwendung in vitro

Periphere mononukleare Blutzellen (PBMCs) aus gesunden Probanden, Melanom- oder MS-Patienten werden von dem Blut mittels Lymphocytentrennmedium (Histopaque, Sigma Aldrich) abgetrennt. Hierzu werden zwei Röhrchen Blut (7 oder 10 ml) von demselben Probanten bzw. Patienten in ein steriles 50 ml Röhrchen überführt und mit RPMI 1640 (InVitrogen, # 14190-69) auf 30 ml aufgefüllt. Anschließend werden 30 ml des verdünnten Blutes auf 15 ml einer Dichtegradientenlösung (Dichte = 1,077; Histopaque, Sigma Aldrich, # 10771) geschichtet.

Nach einer Zentrifugation bei 400 g für 40 min bei 20°C ohne Bremseinsatz werden zwei "Weiße-Blutzellen-Ringe" geerntet und in ein steriles 50 ml-Röhrchen überführt und zweimal mit Phosphatgepufferter Saline (PBS; InVitrogen # 14190-169) gewaschen. Im Falle einer Kontamination mit roten Blutzellen wird eine RBC ("red blood cells") -Lyse durchgeführt; 2 ml RBC-Lyselösung werden zu dem Zellpellet hinzugegeben, unter sanftem Mixen bei Raumtemperatur erfolgt eine Inkubation für 2 min, gefolgt von einem Waschvorgang mit einem großen Volumen von Komplettmedium (RPMI 1640 mit 10 % Fötalem Kälberserum).

Die Anzahl von lebenden Leukocyten wird durch Ausschlussfärbung mittels Trypanblau (InVitrogen # 15250-061) und einem Hämocytometer (FisherBioblock A2759B) bestimmt.

### 1.2 CFSE-Markierung

Nach der Zählung werden die Zellen zweimal in PBS gewaschen und bei einer Konzentration von 1 x 10⁶ Zellen/ml in PBS resuspendiert. CFSE (InVitrogen # C1157) wird in einer Endkonzentration von 0,5 µM hinzugegeben. Nach 10-minütiger Inkubation im Dunkeln bei 37 °C werden die CFSE-markierten Zellen dreimal mit frischem Komplettmedium bei 4 °C gewaschen und bei einer Konzentration von 1 x 10⁶ Zellen/ml in Komplettmedium zur Ausplattierung resuspendiert.

### 1.3 Stimulierung der PBMCs in vitro

Die PBMCs verbleiben entweder unstimuliert oder werden mit hIL-2-Wildtyp (Proleukin) oder hIL-2-N88R (BAY 50-4798; Charge #PR312C008) mit oder ohne einem Pool von synthetischen Peptiden, die sich von den melanomspezifischen Proteinen gp100, TRP-2, MART-1 und Tyrosinase bzw. dem für Multiple Sklerose (MS) spezifischen Protein MOG ableiten, stimuliert, wobei jedes Peptid in einer Endkonzentration von 2,5 µM (Melanompeptide) bzw. 30 µg/ml (MS-Peptid) hinzugegeben wird.

Stimulator und Peptid werden unter den nachfolgenden 23 Bedingungen hinzugegeben:

**Tabelle 1: Bedingungen zur Stimulation der PBMCs**

| Bedingung | Stimulator | Endkonzentration | |
|---|---|---|---|
| 1 | hIL-2-N88R | 10⁻¹¹ M | keine Peptide |
| | (BAY 50-4798; | | |
| | #PR312C008) | | |
| 2 | hIL-2-N88R | 10⁻¹¹ M | Peptide |
| | (BAY 50-4798; | | |
| | #PR312C008) | | |
| 3 | hIL-2-N88R | 10⁻⁹ M | keine Peptide |
| | (BAY 50-4798; | | |
| | #PR312C008) | | |
| 4 | hIL-2-N88R | 10⁻⁹ M | Peptide |
| | (BAY 50-4798; | | |
| | #PR312C008) | | |
| 5 | hIL-2-N88R | 10⁻⁸ M | keine Peptide |
| | (BAY 50-4798; | | |
| | #PR312C008) | | |
| 6 | hIL-2-N88R | 10⁻⁸ M | Peptide |
| | (BAY 50-4798; | | |
| | #PR312C008) | | |
| 7 | hIL-2-N88R | 10⁻⁷ M | keine Peptide |
| | (BAY 50-4798; | | |
| | #PR312C008) | | |
| 8 | hIL-2-N88R | 10⁻⁷ M | Peptide |
| | (BAY 50-4798; | | |
| | #PR312C008) | | |
| 9 | hIL-2-N88R | 10⁻⁶ M | keine Peptide |
| | (BAY 50-4798; | | |
| | #PR312C008) | | |
| 10 | hIL-2-N88R | 10⁻⁶ M | Peptide |
| | (BAY 50-4798; | | |
| | #PR312C008) | | |
| 11 | hIL-2-Wildtyp | 10⁻¹¹ M | keine Peptide |
| | (Proleukin) | | |
| 12 | hIL-2-Wildtyp | 10⁻¹¹ M | Peptide |
| | (Proleukin) | | |
| 13 | hIL-2-Wildtyp | 10⁻⁹ M | keine Peptide |
| | (Proleukin) | | |
| 14 | hIL-2-Wildtyp | 10⁻⁹ M | Peptide |
| | (Proleukin) | | |
| 15 | hIL-2-Wildtyp | 10⁻⁸ M | keine Peptide |
| | (Proleukin) | | |
| 16 | hIL-2-Wildtyp | 10⁻⁸ M | Peptide |
| | (Proleukin) | | |
| 17 | hIL-2-Wildtyp | 10⁻⁷ M | keine Peptide |
| | (Proleukin) | | |
| 18 | hIL-2-Wildtyp | 10⁻⁷ M | Peptide |
| | (Proteukin) | | |
| 19 | hIL-2-Wildtyp | 10⁻⁶ M | keine Peptide |
| | (Proleukin) | | |
| 20 | hIL-2-Wildtyp | 10⁻⁶ M | Peptide |
| | (Proleukin) | | |
| 21 | PHA | 5 µg/ml | |
| 22 | Unstim. | | |
| 23 | Peptid allein | | Peptide |

Anschließend wurden die Zellen für sechs Tage bei 37 °C und einer Atmosphäre mit 5 % CO₂-Gehalt kultiviert.

### Proliferationsassay und Phänotypysierung auf dem FC500-Durchflusszytometer

Die Färbung der Zellen mit fluoreszenzmarkierten Antikörpern auf Zelloberflächenmolekülen ermöglicht die Untersuchung der Proliferation einer spezifischen Untergruppe von Lymphocyten (Memory- und Aktivierungsmarker, vgl. Tab. 2). Die Immunfärbung mit fluorochrommarkierten (PE: Phycoerythrin, ECD: PE-Texas Red, APC: Allophycocyanin, PC7: PE-Cy7) Antikörpern erfolgt vor und nach sechs Tagen der Kultivierung mit den Stimulatoren.

Am sechsten Tag werden die beiden ersten Färbungen (1 und 1iso) mit nicht-CFSE-markierten Zellen durchgeführt (CFSE: Carboxyfluoresceindiacetatsuccinimidylester); die anderen Färbungen erfolgen an CFSE-markierten Zellen.

**Tabelle 2: Färbeschema der PBMCS**

| | **PE** | **ECD** | **APC** | **PC7** |
|---|---|---|---|---|
| 1 | CD25 | CD45 | Foxp3 | CD4 |
| 1iso | CD25 | CD45 | Ratten-IgG2a | CD4 |
| 2 | CD127 | CD45 | CD25 | CD4 |
| 3 | CD3 | CD45 | CD25 | CD8 |
| 4 | CD16 | | CD56 | CD3 |
| 5 | CCR7 | CD3 | CD45RA | CD4 |
| 6 | CCR7 | CD3 | CD45RA | CD8 |
| 7 | CD8 | CD3 | CD45RO | CD4 |
| | **PE** | **ECD** | **APC** | **PC7** |
| 1 | CD25 | CD3 | Foxp33 | CD4 |
| 1iso | CD25 | CD3 | Ratten-IgG2a | CD4 |
| 2 | CD8 | CD3 | CD25 | CD4 |
| 3 | CCR7 | CD3 | CD45RA | CD4 |
| 4 | CD8 | CD3 | CD45RO | CD4 |

CD25-PE, Foxp3-APC und Ratten-IgG2a-APC stammen von ebiosciences; CD25-APC, CD45RA-APC und CD45RO-APC wurden bezogen von BD Biosciences. Sämtliche anderen Antikörper stammen von Beckman-Coulter, Frankreich.

### 1.5 Maus- Diabetes-Typ-I-Modell

12 Wochen alte NOD ("Non-obese diabetes") Mäuse werden täglich mit hIL-2-Mutein oder hIL-2 Wildtyp behandelt. Negativ-Kontrolltiere wurden analog mit physiologischer Salzlösung (Saline) behandelt. Die Behandlungsgruppen bestanden aus 3 - 5 Tieren. An Tag 0 bis 15 wurde den Mäusen eine Menge von 5K- oder 25K-Einheiten hIL-2-Mutein oder hIL-2 Wildtyp appliziert. Ab Tag 17 wurde in den Behandlungsgruppen mit 5K-Einheiten auf 100K-Einheiten (= 6,112 µg) erhöht. Die Behandlung der anderen Tiere mit 25K-Einheiten wurde unverändert beibehalten. Die letzte Dosierung wurde an Tag 31 vorgenommen. In einem parallelen Experiment wurde von Tag 0 bis Tag 31 mit einer festen Dosis von 25K-Einheiten behandelt. Diabetes wurde mittels Monitorierung von Glukosespiegeln im Urin nachgewiesen. Den Mäusen wurden an Tag 17 und Tag 30 Blutproben abgenommen. Analysiert wurden die Proben im FACS mittels Anti-CD4-, Anti-CD25- sowie Anti-FoxP3-Färbung und somit wurde der prozentuale Anteil der FoxP3⁺-Zellen unter den CD4⁺-T-Zellen sowie die mittlere Fluoreszenzintensität (MFI) der CD25-Expression auf CD4⁺FoxP3⁺-Zellen bestimmt.

### 2. Ergebnisse

### 2.1 Induktion von Regulatorischen T-Zellen durch hIL-2-N88R

Als geeignetes *In-vivo-*System, um den Effekt der erfindungsgemäßen Muteine zu testen, wurden Periphere mononukleare Blutzellen (PBMCs) verwendet. PBMCs bestehen aus T-Zellen (- 75 % CD4- und CD8-positiv) und B- und NK-Zellen (~ 2S % positiv) und bilden somit eine das Immunsytem gut repräsentierende Zellpopulation.

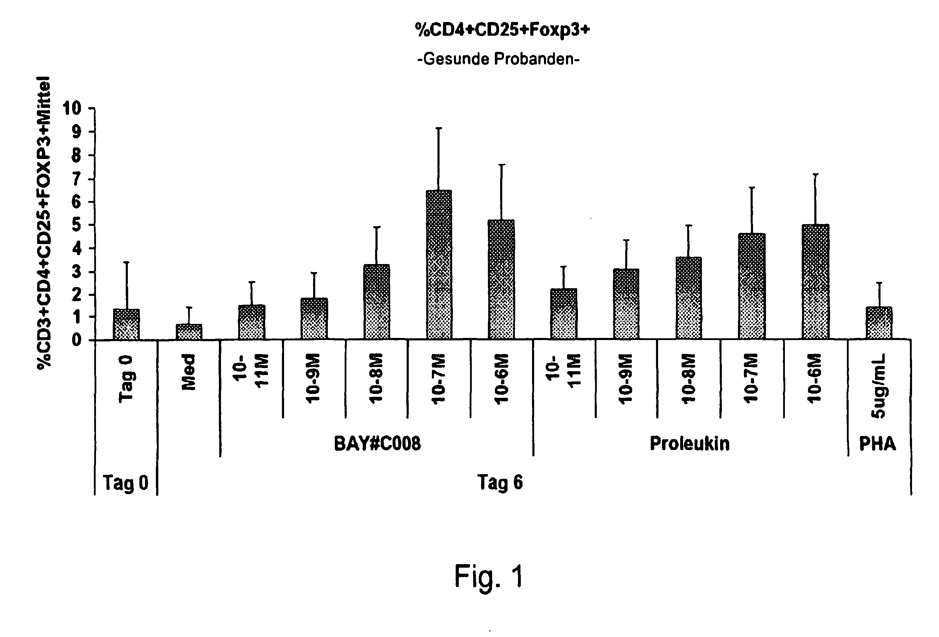

PBMCs aus sechs gesunden Probanden (10⁶ Zellen/ml) wurden mit Wildtyp-IL-2 (Proleukin) oder IL-2-N88R [BAY 50-4798, Charge #PR312C008 ("BAY#C008")] bei Konzentrationen, die zwischen 10⁻¹¹ und 10⁻⁶ M lagen, oder in der Positivkontrolle mit dem unspezifischen Mitogen Phytohämagglutinin ("PHA") bei einer Konzentration von 5 µg/ml oder mit Kulturmedium allein ("Med") stimuliert. Am Tag 0 und am sechsten Tag nach der Stimulation wurde der Anteil der Regulatorischen CD4⁺CD25⁺Foxp3⁺-T-Zellen innerhalb der CD3⁺-Lymphocyten bestimmt. Das Ergebnis ist in der Fig. 1 und der nachfolgenden Tabelle 3 dargestellt.

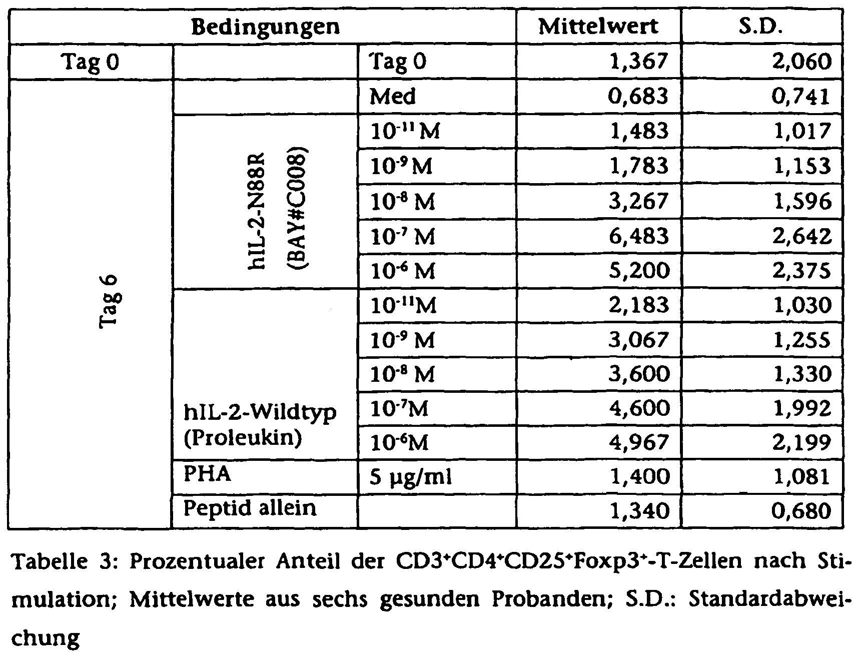

Aus diesem Experiment geht hervor, dass hIL-2-N88R bei Konzentrationen von 10⁻⁷ M und 10⁻⁶ M zu einer deutlichen Induktion der Subpopulation der Regulatorischen T-Zellen CD4⁺CD25⁺Foxp3⁺ führt. Die Induktion ist dabei deutlich größer als bei einer Stimulation der PBMCs durch hIL-2-Wildtyp.

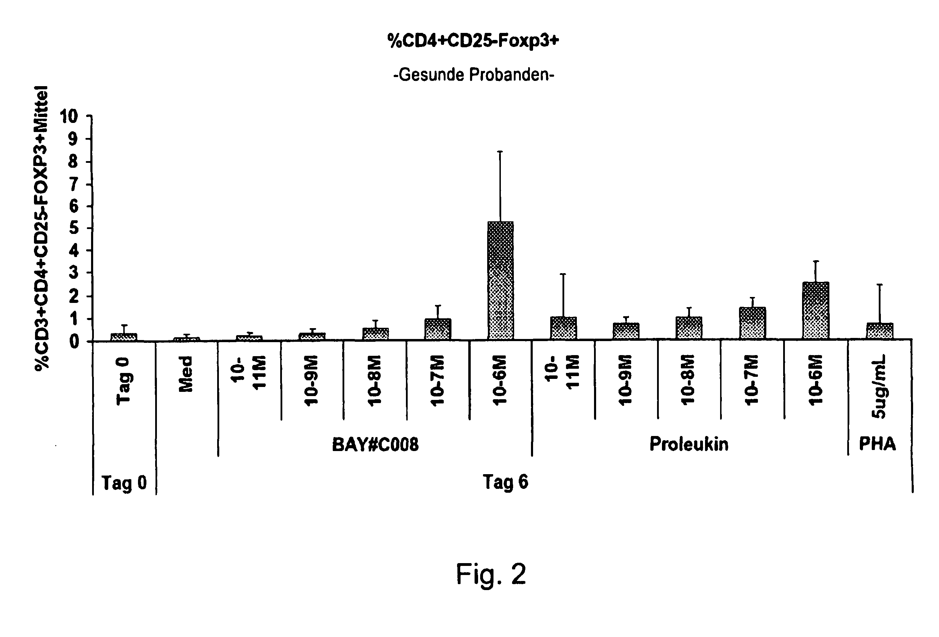

In einem zweiten Ansatz wurde der Anstieg der Subpopulation der Regulatorischen T-Zellen CD4⁺CD25⁻Foxp3⁺ nach Stimulation mit hIL-2-N88R im Vergleich zu hIL-2-Wildtyp untersucht. Das Ergebnis ist in der Fig. 2 bzw. der nachfolgenden Tabelle 4 dargestellt.

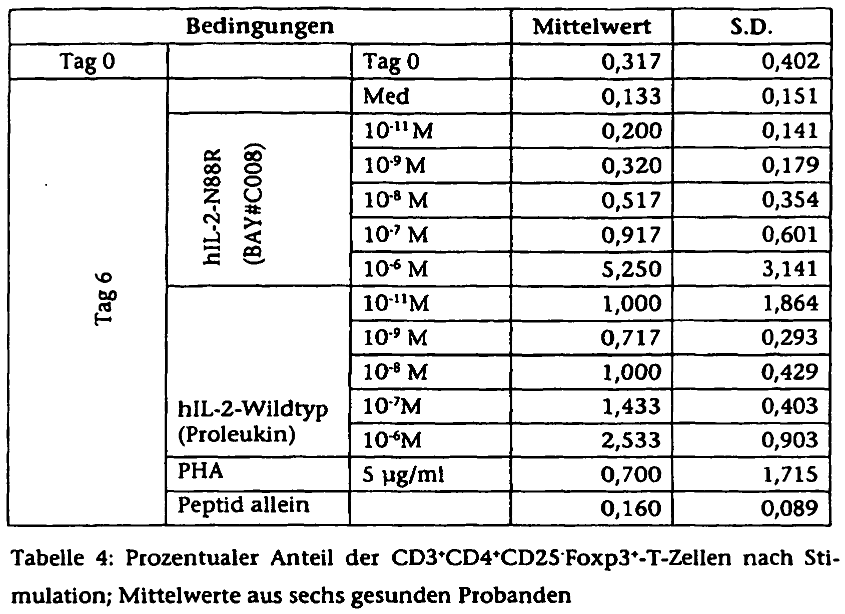

Auch hier zeigt sich, dass die Stimulation mit hIL-2-N88R zu einem deutlichen Anstieg der Subpopulation der Regulatorischen T-Zellen CD4⁺CD25⁻Foxp3⁺ führt, der bei Konzentrationen von 10⁻⁶ M deutlich größer ist als bei einer Stimulation mit hIL-2-Wildtyp.

### 2.2 hIL-2-N88R induziert Regulatorische T-Zellen in Melanompatieten

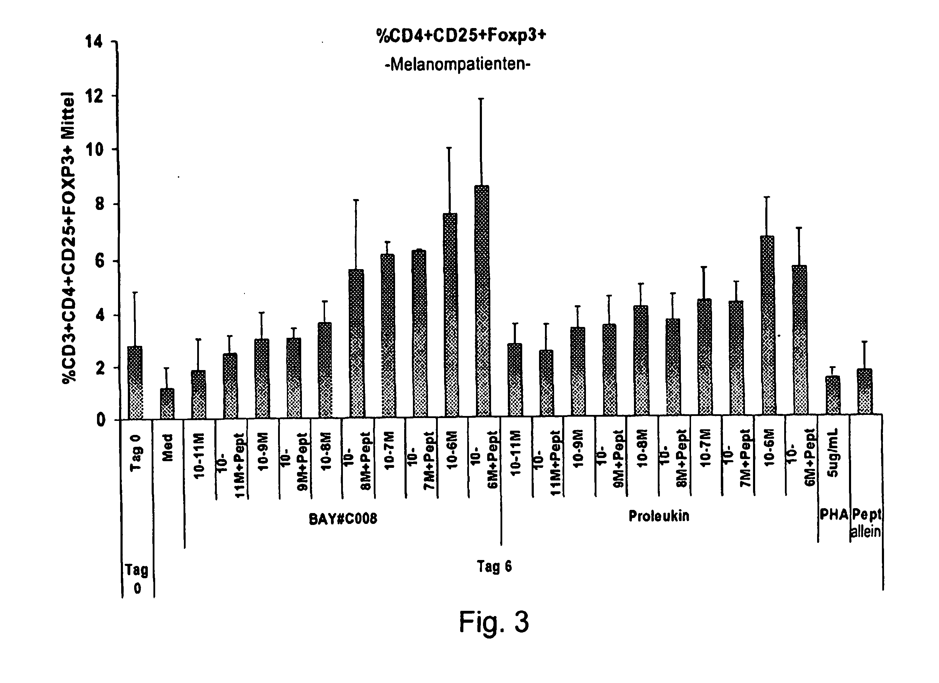

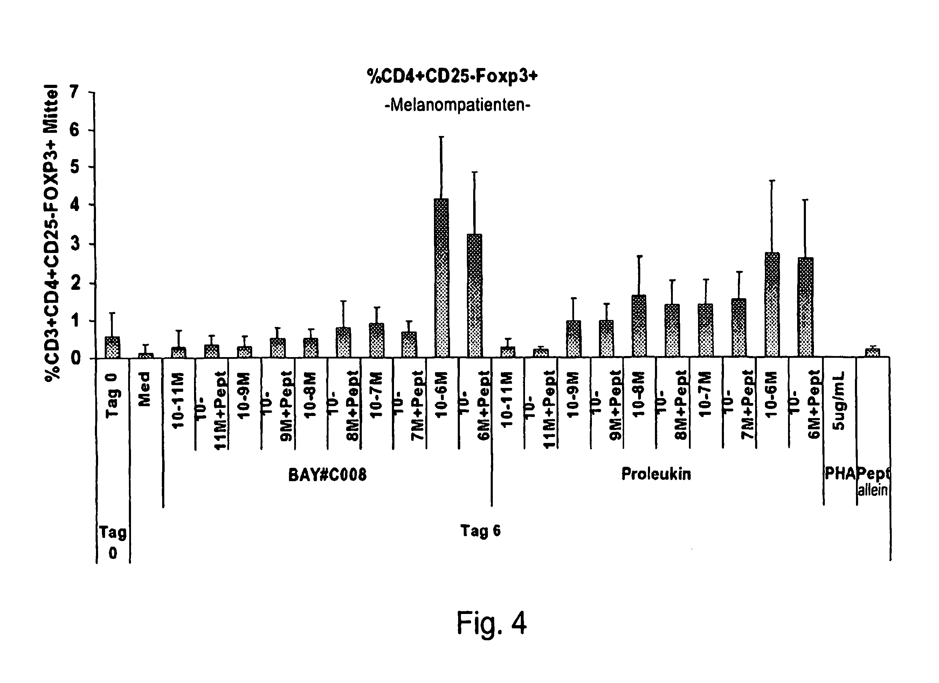

Als nächstes wurde untersucht, ob das erfindungsgemäße hIL-2-Mutein N88R auch die antigenspezifische Aktivität von Immunzellen stimuliert. Hierzu wurden PBMCs (10⁶ Zellen/ml) aus drei Melanompatienten mit hIL-2-N88R (BAY 50-4798, Charge #PR312C008) oder hIL-2-Wildtyp (Proleukin) bei Konzentrationen, die zwischen 10⁻¹¹ und 10⁻⁶ M lagen, in Gegenwart oder Abwesenheit eines melanomassoziierten Peptidpools, mit 5 µg/ml PHA oder mit Kulturmedium allein stimuliert. Anschließend wurden die Subpopulationen der Regulatorischen T-Zellen CD4⁺CD25⁺Foxp3⁺ bzw. CD4⁺CD25⁻Foxp3⁺ bestimmt. Das Ergebnis ist in der Fig. 3 und Tabelle 5 bzw. Fig. 4 und Tabelle 6 dargestellt.

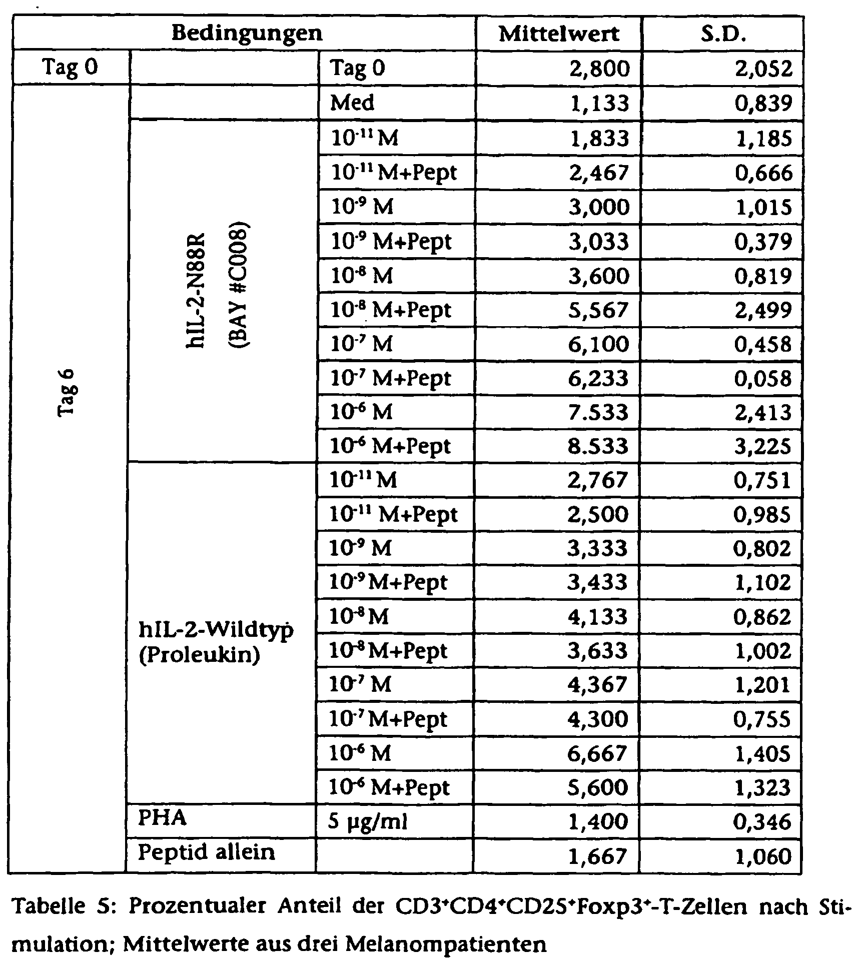

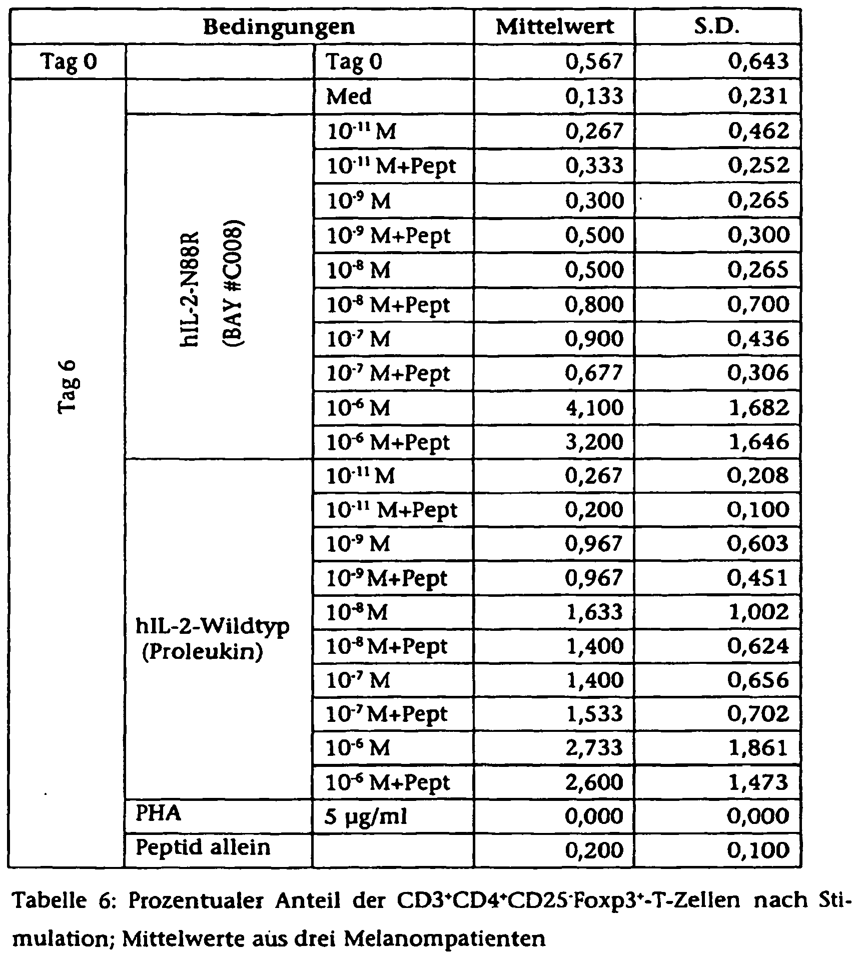

Dabei zeigte sich, dass die Verabreichung von hIL-2-88R auch in Melanompatienten zu einem deutlichen Anstieg der Regulatorischen T-Zellen führt. Dieser ist im Falle der Subpopulation CD4⁺CD25⁺Foxp3⁺ bei Konzentration von 10⁻⁷ M und 10⁻⁶ M, und bei der Subpopulation CD4⁺CD25⁻Foxp3⁺ bei einer Konzentration von 10⁻⁶ M deutlich größer als bei einer Stimulation mit entsprechenden Konzentrationen von Wildtyp-IL-2 (Proleukin).

### 2.3 hIL-2-N88R induziert Regulatorische T-Zellen in Patienten mit Multipler Sklerose

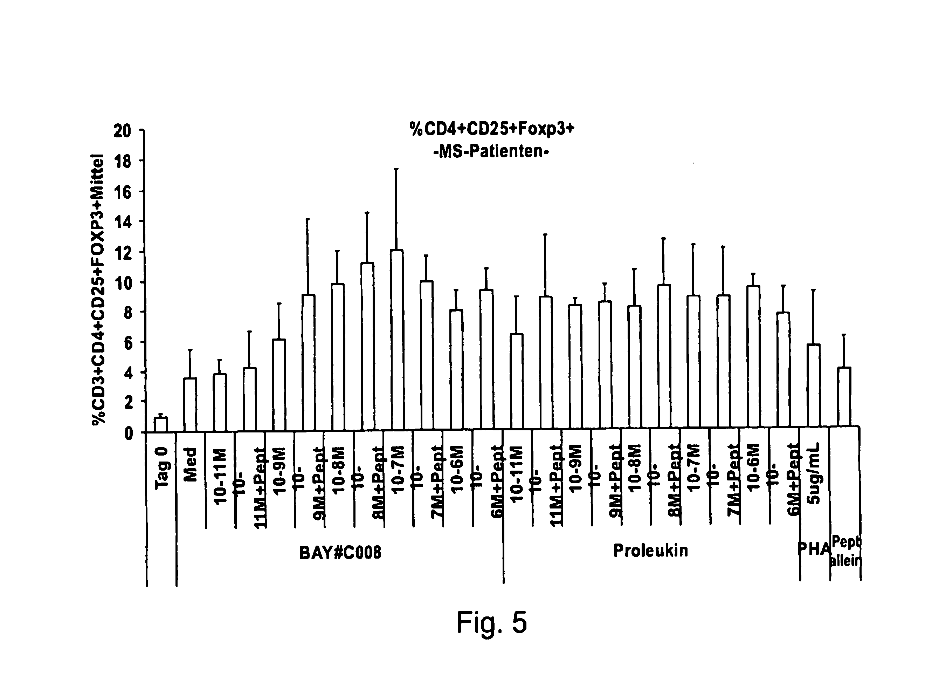

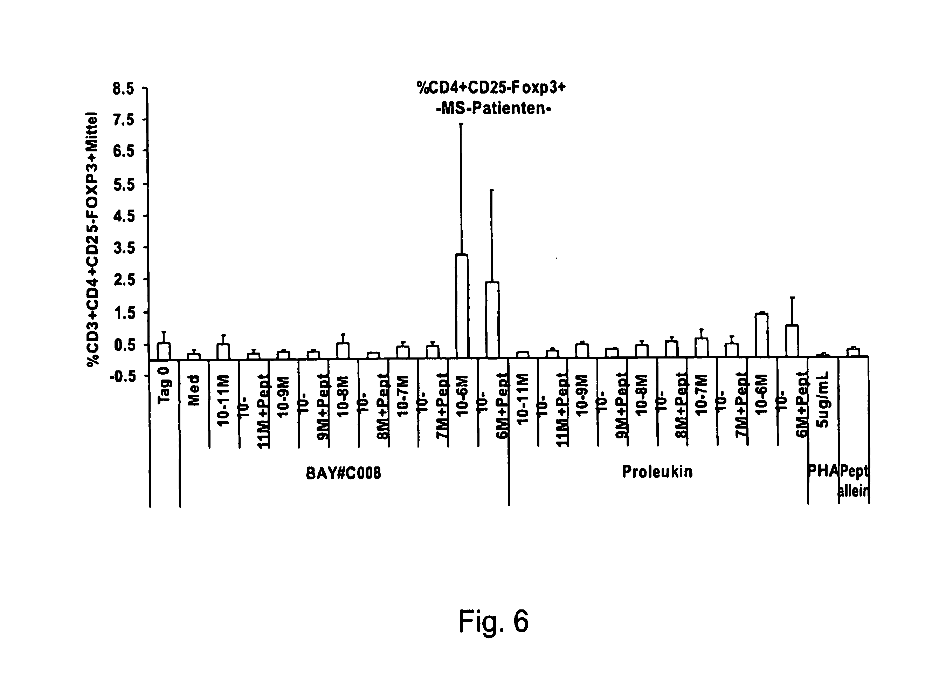

Als nächstes wurde untersucht, ob das erfindungsgemäße hIL-2-Mutein N88R auch die antigenspezifische Aktivität von Immunzellen stimuliert. Hierzu wurden PBMCs (10⁶ Zellen/ml) aus zwei Multiple-Sklerose-Patienten mit hIL-2-N88R (BAY 50-4798, Charge #PR312C008) oder hIL-2-Wildtyp (Proleukin) bei Konzentrationen, die zwischen 10⁻¹¹ und 10⁻⁶ M lagen, in Gegenwart oder Abwesenheit eines Multiple-Sklerose-assoziierten Peptids, mit 5 µg/ml PHA oder mit Kulturmedium allein stimuliert. Anschließend wurden die Subpopulationen der Regulatorischen T-Zellen CD4⁺CD25⁺Foxp3⁺ bzw. CD4⁺CD25⁻Foxp3⁺ bestimmt. Das Ergebnis ist in der Fig. 5 und Tabelle 7 bzw. Fig. 6 und Tabelle 8 dargestellt.

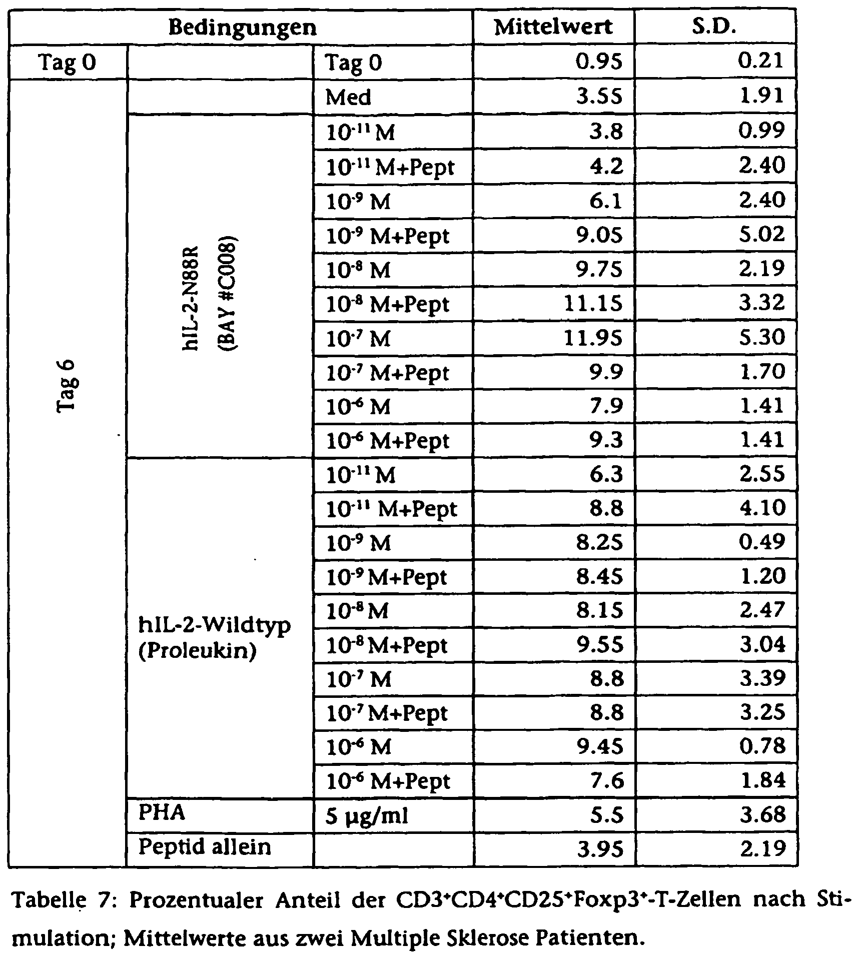

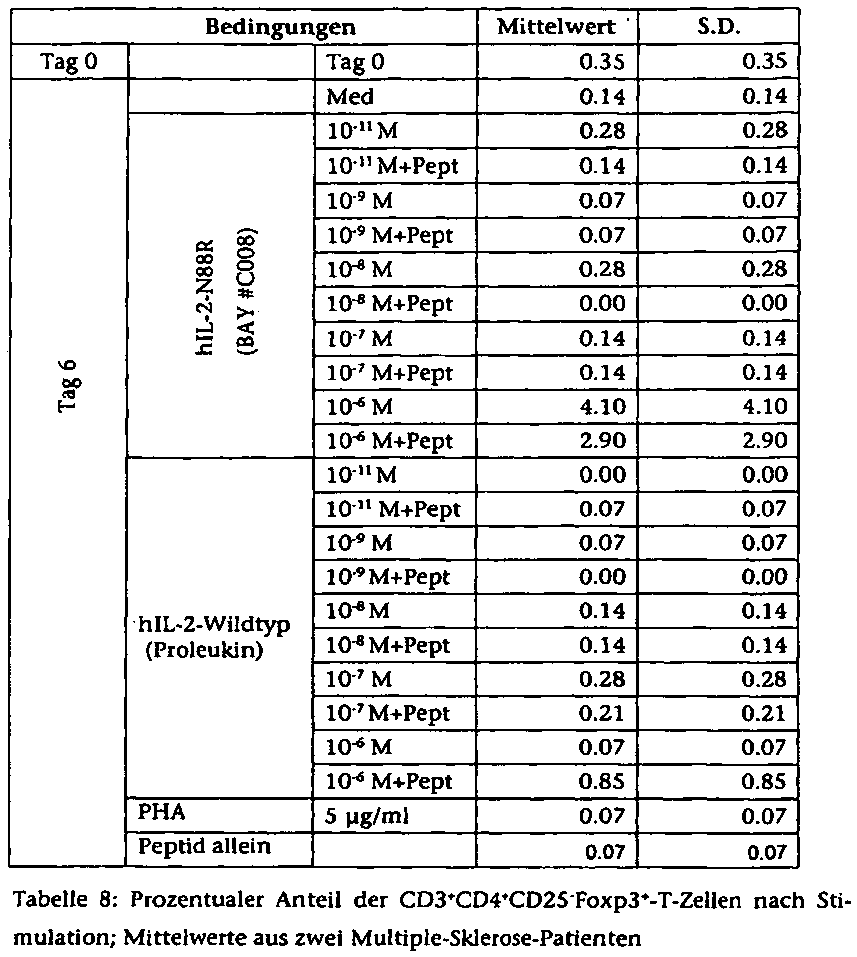

Es zeigte sich, dass die Verabreichung von hIL-2-88R auch in Multiple-Sklerose-Patienten zu einem deutlichen Anstieg der Regulatorischen T-Zellen führt. Dieser ist im Falle der Subpopulation CD4⁺CD25⁺Foxp3⁺ bei Konzentration von 10⁻⁸ M und 10⁻⁷ M, und bei der Subpopulation CD4⁺CD25⁻Foxp3⁺ bei einer Konzentration von 10⁻⁶ M deutlich größer als bei einer Stimulation mit entsprechenden Konzentrationen von Wildtyp-IL-2 (Proleukin).

### 2.4 hIL-2-N88R induziert nur minimale Proliferation von cytotoxischen CD8⁺-T-Zellen in Patienten mit Multipler Sklerose und in gesunden Probanden

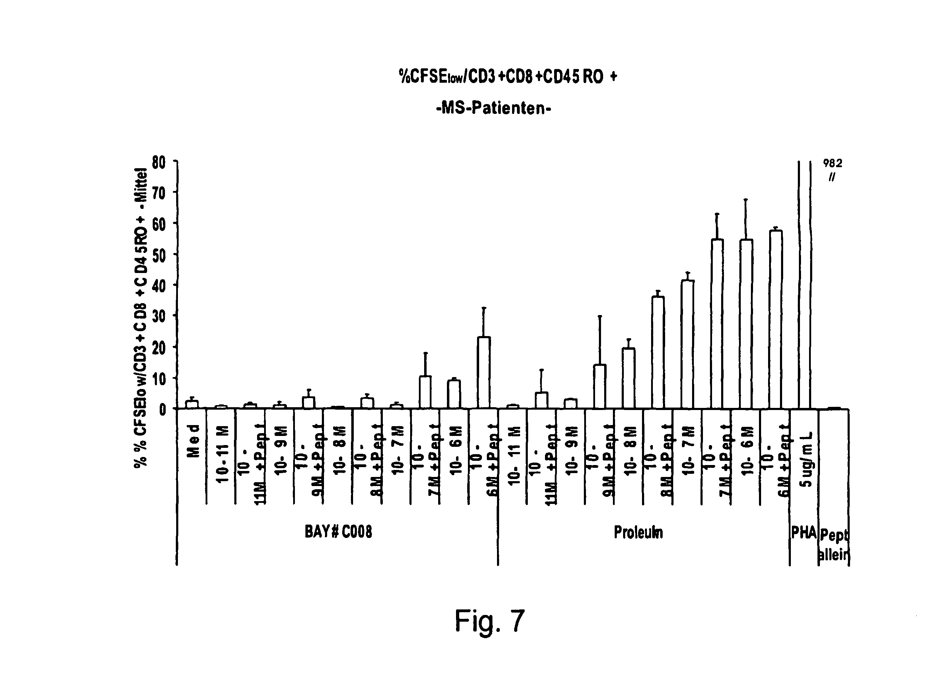

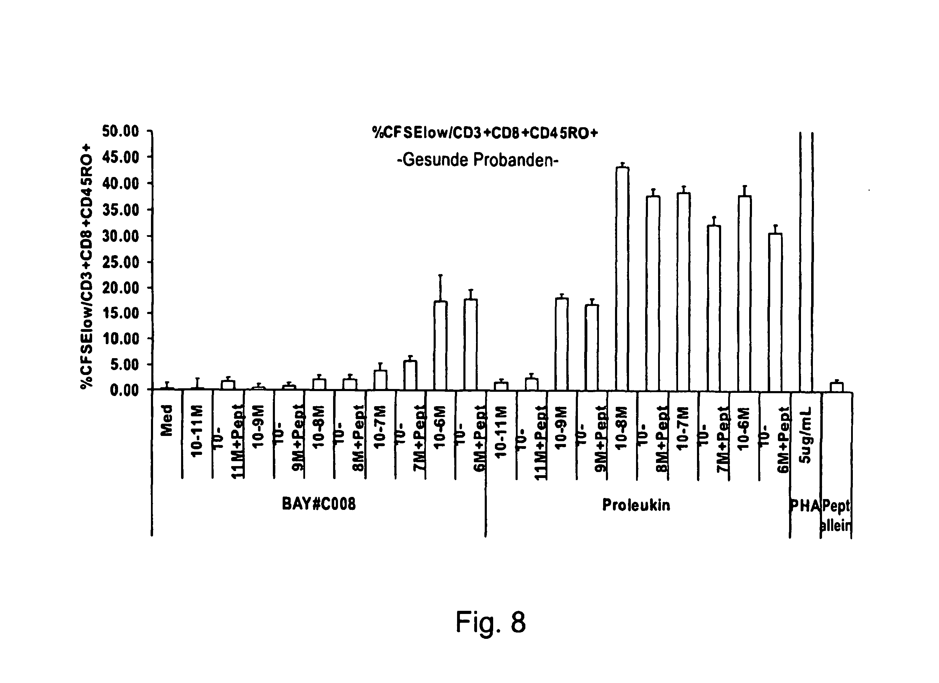

Weiterhin wurde die Stimulation von cytotoxischen CD8+ central memory T-Zellen untersucht. Dazu wurden PBMCs von gesunden Probanden oder Multiple-Sklerose-Patienten, wie unter 2.3 beschrieben, behandelt. Analysiert wurde der prozentuale Anteil der CFSElow/CD3⁺CD8⁺CD45RO⁺-T-Zellen. Das Ergebnis ist in der Fig. 7 und Tabelle 9 sowie Fig. 8 und Tabelle 10 dargestellt.

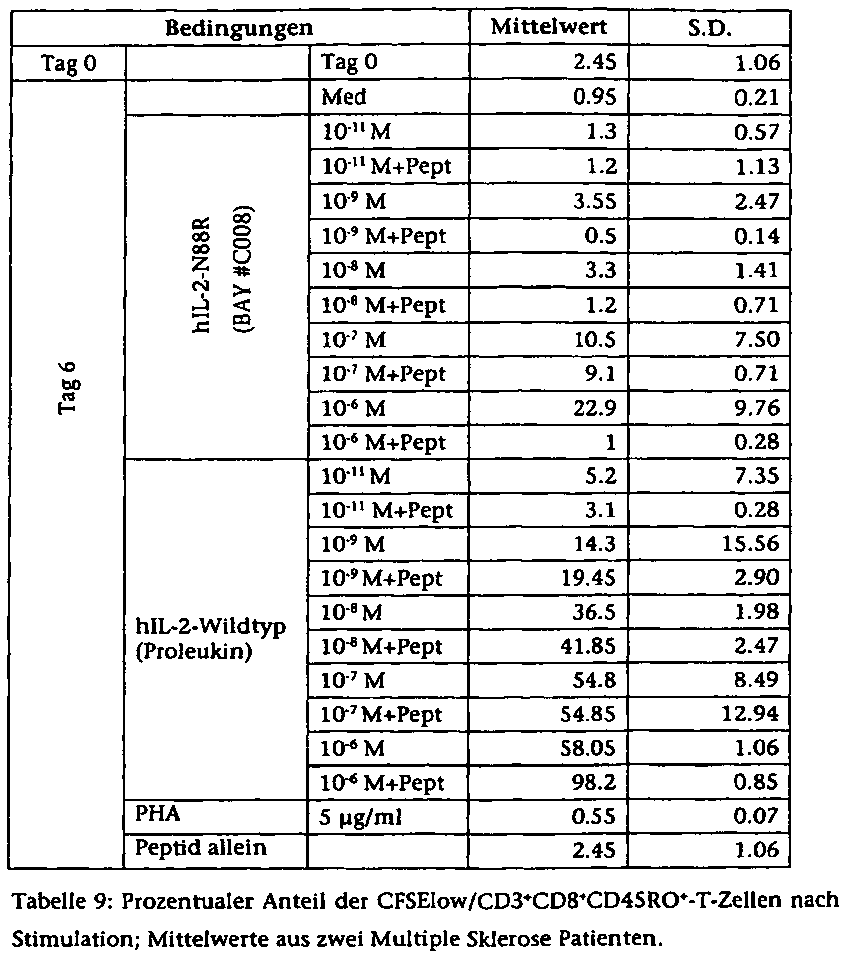

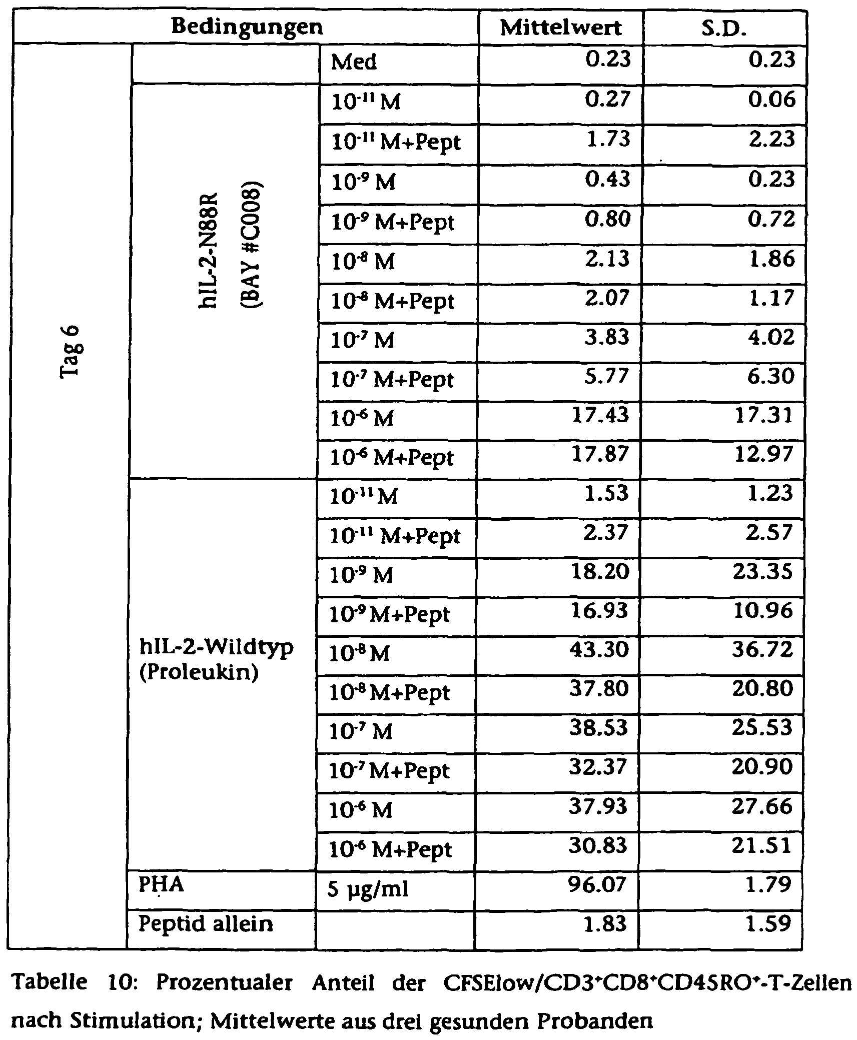

hIL-2-88-R führt in Multiple Sklerose Patienten als auch in gesunden Probanden im Gegensatz zu hIL-2-Wildtyp zu einer nur geringfügigen Proliferation von central memory CD8⁺ T-Zellen, und zwar in jeder untersuchten Konzentration.

### 2.5 Behandlung mit hIL-2-Mutein verhindert die Entwicklung von Diabetes typ I im Tiermodel

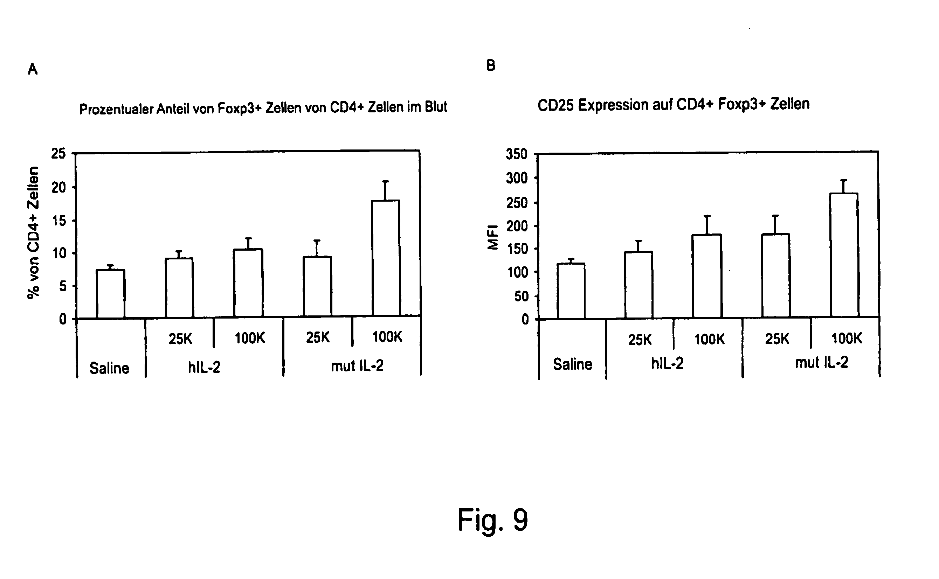

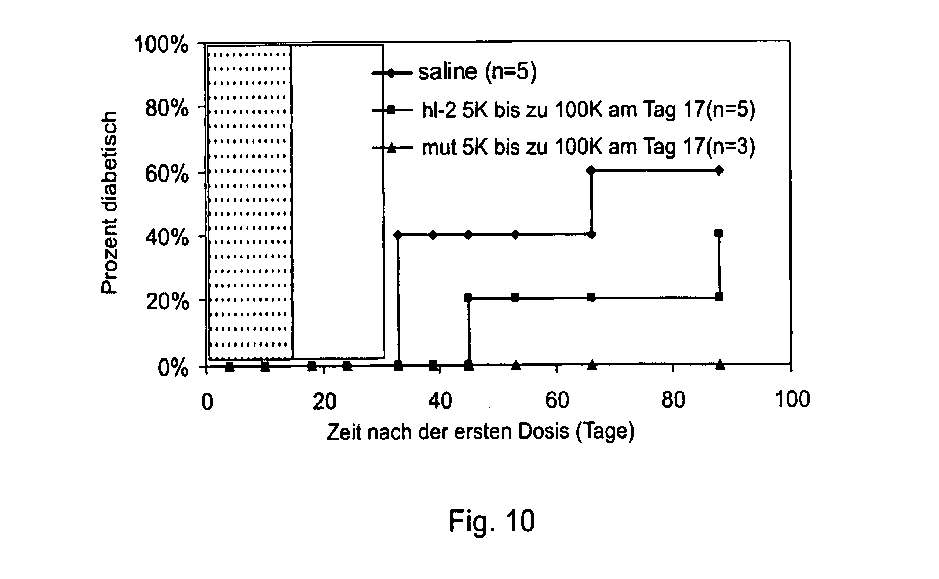

Die Behandlung von NOD Mäusen mit hIL-2-N88R führt im Vergleich zu hIL-2 Wildtyp zu einem höheren prozentualen Anstieg von FoxP3+ Zellen innerhalb der CD4+ Zellen (Fig. 9 (A)). Diese CD4+FoxP3+ positiven Zellen weisen außerdem eine höhere Expression von CD25 auf (Fig. 9 (B)). Fig. 10 zeigt, dass hIL-2-N88R Behandlung im Maus Diabetes Typ I Modell im Gegensatz zum hIL-2-Wildtyp die Entwicklung der Diabetes in allen Mäusen der Behandlungsgruppe unterbindet.

### 3. Fazit

Die von den Erfindern durchgeführten Experimente verdeutlichen, dass es sich bei den erfindungsgemäßen hIL-2-Muteinen und Abschnitten hiervon aufgrund ihres Potentials zur Induktion von Regulatorischen T-Zellen (T_{Reg}) um Substanzen handelt, die zur Behandlung und/oder Prophylaxe einer Autoimmunerkrankung bzw. zur Induktion von T_{Reg} in einem Lebewesen sowie zur zur Bildung von T_{Reg} *in vitro,* geeignet sind. Dies wird von den Erfindern nicht nur *in vitro* sondern auch *in vivo* gezeigt.

## Patentansprüche

1. Verwendung eines Muteins von humanem Interleukin-2 (hIL-2-Mutein), das entsprechend dem hIL-2-Wildtyp nummeriert ist und eine Aminosäuresubstitution an zumindest einer der Positionen 20, 88 oder 126 aufweist, zur Herstellung eines Arzneimittels zur Behandlung und/oder Prophylaxe einer Autoimmunerkrankung, **dadurch gekennzeichnet, dass**
- durch die Substitution an Position 88 ein Asparagin gegen ein Arginin (hIL-2-N88R), oder gegen ein Glycin (hIL-2-N88G), oder gegen ein Isoleucin (hIL-2-N88I) ausgetauscht ist,
- durch die Substitution an Position 20 eine Asparaginsäure gegen ein Histidin (hIL-2-D20H), oder gegen ein Isoleucin (hIL-2-D20I), oder gegen ein Tyrosin (hIL-2-D20Y) ausgetauscht ist, und
- durch die Substitution an Position 126 ein Glutamin gegen ein Leucin (hIL-2-Q126L) ausgetauscht ist.

2. Verwendung nach Anspruch 1, **dadurch gekennzeichnet, dass** das Arzneimittel zusätzlich ein Immunsuppressivum aufweist.

3. Verwendung nach Anspruch 2, **dadurch gekennzeichnet, dass** das Immunsuppressivum ausgewählt ist aus der Gruppe bestehend aus: Glukokortikoid, einschließlich Decortin, Prednisol; Azathioprin; Cyclosporin A; Mykophenolatmofetil; Tacrolimus; Anti-T-Lymphocytenglobulin, Anti-CD3-Antikörper, einschließlich Muromonab; Anti-CD25-Antikörper, einschließlich Basiliximab und Daclizumab; Anti-TNF-α-Antikörper, einschließlich Infliximab und Adalimumab; Azathioprin; Methotrexat; Ciclosporin; Sirolimus; Everolimus; Fingolimod; CellCept; Myfortic; Cyclophosphamid.

4. Verwendung nach einem der vorherigen Ansprüche, **dadurch gekennzeichnet, dass** die Autoimmunerkrankung ausgewählt ist aus der Gruppe bestehend aus:
Diabetes-mellitus-Typ I, Rheumatoide Arthritis, Multiple Sklerose, Chronische Gastritis, Morbus Crohn, Morbus Basedow, Morbus Bechterew, Psoriasis, Myasthenia gravis, Autoimmunhepatitis, APECED, Chrug-Strauss-Syndrom, Colitis ulcerosa, Glomerulonephritis, Guillain-Barré-Syndrom, Hashimoto-Thyreoiditis, Lichen sclerolus, systemischer Lupus erythematodes, PANDAS, Rheumatisches Fieber, Sarkoidose, Sjörgren-Syndrom, Stiff-Man-Syndrom, Sklerodermie, Wegenersche Granulomatose, Vitilogo, Autoimmunenteropathie, Goodpasture-Syndrom, Dermatomyositis, Polymyositis, Autoimmunallergie, Asthma und Autoimmunreaktion nach Organtransplantationen.

5. Verwendung nach einem der vorherigen Ansprüche, **dadurch gekennzeichnet, dass** das Arzneimittel einen pharmazeutisch akzeptablen Träger aufweist.

6. Arzneimittel zur Behandlung und/oder Prophylaxe einer Autoimmunerkrankung, **dadurch gekennzeichnet, dass** es das hIL-2-Mutein gemäß der Verwendung nach einem der Ansprüche **1** bis 5 aufweist.

## Claims

1. Use of a mutein of human interleukin-2 (hIL-2 mutein), which is numbered correspondingly to the hIL-2 wild type and comprises an amino acid substitution in at least one of the positions 20, 88 or 126, for the preparation of a medicament for the treatment and/or prophylaxis of an autoimmune disease, **characterized in that**
- through the substitution at position 88 an asparagine is exchanged for an arginine (hIL-2-N88R), or for a glycine (hIL-2-N88G), or for an isoleucine (hIL-2-N88I),
- through the substitution at position 20 an aspartic acid is exchanged for a histidine (hIL-2-D20H), or for an isoleucine (hIL-2-D20I), or for a tyrosine (hIL-2-D20Y), and
- through the substitution at position 126, a glutamine is exchanged for a leucine (hIL-2-Q126L).

2. Use according to claim 1, **characterized in that** the medicament in addition comprises an immunosuppressant.

3. Use according to claim 2, wherein the immunosuppressant is selected from the group consisting of: glucocorticoid, including decortin, prednisol; azathioprine; cyclosporin A; mycophenolate mofetil; tacrolimus; anti-T-lymphocyte globulin, anti-CD3-antibodies, including muromonab; anti-CD25-antibodies, including basiliximab and daclizumab; anti-TNF-α antibodies, including infliximab and adalimumab; azathioprine; methotrexate; cyclosporin; sirolimus; everolimus; fingolimod; CellCept; myfortic; and cyclophosphamide.

4. Use according to any of the previous claims, wherein the autoimmune disease is selected from the group consisting of: type I diabetes mellitus, rheumatoid arthritis, multiple sclerosis, chronic gastritis, Crohn's disease, Basedow disease, Bechterew disease, psoriasis, myasthenia gravis, autoimmune hepatitis, APECED, Chrug-Strauss syndrome, ulcerative colitis, glomerulonephritis, Guillain-Barré syndrome, Hashimoto thyroiditis, lichen sclerosus, systemic lupus erythematodes, PANDAS, rheumatic fever, sarcoidosis, Sjörgren syndrome, Stiff-Man syndrome, scleroderma, Wegener's granulomatosis, vitiligo, auto-immune enteropathy, Goodpasture syndrome, dermatomyositis, polymyositis, autoimmune allergy, asthma and autoimmune reaction after organ transplantations.

5. Use according to any of the previous claims, wherein the agent contains a pharmaceutically acceptable carrier.

6. Pharmaceutical composition for the treatment and/or prophylaxis of an autoimmune disease, wherein it contains the hIL-2 mutein or a fragment thereof according to the use according to one of claims 1 to 5.

## Revendications

1. Utilisation d'une mutéine d'interleukine 2 humaine (mutéine hIL-2) qui est numérotée de façon correspondant à hIL-2 de type sauvage et présente une substitution d'acides aminés sur au moins l'une des positions 20, 88 ou 126, pour la production d'un médicament destiné au traitement et/ou à la prophylaxie d'une maladie auto-immune, **caractérisée en ce que**
- par la substitution en position 88, une asparagine est échangée contre une arginine (hIL-2-N88R) ou contre une glycine (hIL-2-N88G) ou contre une isoleucine (hIL-2-N88I),
- par la substitution en position 20, un acide aspartique est échangé contre une histidine (hIL-2-D20H) ou contre une isoleucine (hIL-2-D20I) ou contre une tyrosine (hIL-2-D20Y), et
- par la substitution en position 126, une glutamine est échangée contre une leucine (hIL-2-Q126L).

2. Utilisation selon la revendication 1, **caractérisée en ce que** le médicament présente en outre un immunosuppresseur.

3. Utilisation selon la revendication 2, **caractérisée en ce que** l'immunosuppresseur est choisi dans le groupe comprenant: un glucocorticoïde comprenant la décortine, le prednisol ; l'azathioprine ; la cyclosporine A ; le mycophénolatemofétil, le tacrolimus ; la globuline anti-lymphocytes T, les anticorps anti-CD3 comprenant le muromonab ; les anticorps anti-CD25 comprenant le basiliximab et le daclizumab ; les anticorps anti-TNF-alpha comprenant l'infliximab et l'adalimumab; l'azathioprine ; le méthotrexate ; la ciclosporine ; le sirolimus ; l'éverolimus ; le fingolimod ; le CellCept ; le myfortic ; le cyclophosphamide.

4. Utilisation selon l'une des revendications précédentes, **caractérisée en ce que** la maladie auto-immune est choisie dans le groupe comprenant : le diabète sucré de type I, l'arthrite rhumatoïde, la sclérose en plaques, la gastrite chronique, la maladie de Crohn, la maladie de Basedow, la maladie de Bechterew, le psoriasis, la myasthénie grave, l'hépatite auto-immune, le syndrome APECED, le syndrome de Churg et Strauss, la rectocolite hémorragique, la glomérulonéphrite, le syndrome de Guillain-Barré, la thyroïdite d'Hashimoto, le lichen scléreux, le lupus érythémateux systémique, le syndrome de PANDAS, la fièvre rhumatoïde, la sarcoïdose, le syndrome de Sjôrgren, le syndrome de l'homme raide ("stiff-man"), la sclérodermie, la granulomatose de Wegener, le vitiligo, l'entéropathie auto-immune, le syndrome de Goodpasture, la dermatomyosite, la polymyosite, les allergies auto-immunes, l'asthme et les réactions auto-immunes consécutives à des greffes d'organe.

5. Utilisation selon l'une des revendications précédentes, **caractérisée en ce que** le médicament présente un véhicule pharmaceutiquement acceptable.

6. Médicament destiné au traitement et/ou à la prophylaxie d'une maladie auto-immune, **caractérisé en ce qu'**il présente la mutéine hIL-2 de l'invention, selon l'une des revendications 1 à 5.


### Sequenzprotokoll

<110> AiCuris GmbH & Co. KG Wuppertal, Deutschland
<120> Mittel zur Behandlung und/oder Prophylaxe einer Autoimmunerkrankung und zur Bildung von Regulatorischen T-Zellen
<130> 1043P108
<160> 3
<170> PatentIn Ver. 2.0
<210> 1
<211> 133
<212> PRT
<213> Homo sapiens
<400> 1
<210> 2
<211> 465
<212> DNA
<213> Homo sapiens
<400> 2
   atgtacagga tgcaactcct gtcttgcatt gcactaagtc ttgcacttgt cacaaacagt 60
   gcacctactt caagttctac aaagaaaaca cagctacaac tggagcattt actgctggat 120
   ttacagatga ttttgaatgg aattaataat tacaagaatc ccaaactcac caggatgctc 180
   acatttaagt tttacatgcc caagaaggcc acagaactga aacatcttca gtgtctagaa 240
   gaagaactca aacctctgga ggaagtgcta aatttagctc aaagcaaaaa ctttcactta 300
   agacccaggg acttaatcag caatatcaac gtaatagttc tggaactaaa gggatctgaa 360
   acaacattca tgtgtgaata tgctgatgag acagcaacca ttgtagaatt tctgaacaga 420
   tggattacct tttgtcaaag catcatctca acactgactt gataa 465
<210> 3
<211> 133
<212> PRT
<213> Künstliche Sequenz
<220> MUTAGEN
<223> hIL-2-N88R
<400> 3
   Ala Pro Thr Ser Ser Ser Thr Lys Lys Thr Gln Leu Gln Leu Glu His
   1 5 10 15
   Leu Leu Leu Asp Leu Gln Met Ile Leu Asn Gly Ile Asn Asn Tyr Lys
   20 25 30
   Asn Pro Lys Leu Thr Arg Met Leu Thr Phe Lys Phe Tyr Met Pro Lys
   35 40 45
   Lys Ala Thr Glu Leu Lys His Leu Gln Cys Leu Glu Glu Glu Leu Lys
   50 55 60
   Pro Leu Glu Glu Val Leu Asn Leu Ala Gln Ser Lys Asn Phe His Leu
   65 70 75 80
   Arg Pro Arg Asp Leu Ile Ser Arg Ile Asn Val Ile Val Leu Glu Leu
   85 90 95
   Lys Gly Ser Glu Thr Thr Phe Met Cys Glu Tyr Ala Asp Glu Thr Ala
   100 105 110
   Thr Ile Val Glu Phe Leu Asn Arg Trp Ile Thr Phe Cys Gln Ser Ile
   115 120 125
   Ile Ser Thr Leu Thr
   130